(19) Europäisches Patentamt
European Patent Office
Office européen des brevets

(11) EP 3 730 510 A1

# (12) EUROPEAN PATENT APPLICATION

(43) Date of publication:
**28.10.2020 Bulletin 2020/44**

(21) Application number: **20176384.4**

(22) Date of filing: **12.03.2013**

(51) Int Cl.:
*C07K 1/16* (2006.01) *B01D 15/36* (2006.01)
*B01J 20/26* (2006.01) *B01J 20/32* (2006.01)
*C07K 1/18* (2006.01) *C08F 220/58* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2012 US 201261609533 P**
**29.06.2012 US 201261666578 P**

(62) Document number(s) of the earlier application(s) in accordance with Art. 76 EPC:
**17188443.0 / 3 312 190**
**13275053.0 / 2 639 239**

(71) Applicant: **Merck Patent GmbH**
**64293 Darmstadt (DE)**

(72) Inventors:
- **KOZLOV, Mikhail**
**Burlington, MA Massachusetts 01803 (US)**
- **CATALDO, William**
**Burlington, MA Massachusetts 01803 (US)**
- **POTTY, Ajish**
**Burlington, MA Massachusetts 01803 (US)**
- **GALIPEAU, Kevin**
**Burlington, MA Massachusetts 01803 (US)**
- **HAMZIK, James**
**Burlington, MA Massachusetts 01803 (US)**
- **UMANA, Joaquin**
**Burlington, MA Massachusetts 01803 (US)**
- **PEECK, Lars**
**Burlington, MA Massachusetts 01803 (US)**

(74) Representative: **Graham Watt & Co LLP**
**St. Botolph's House**
**7-9 St. Botolph's Road**
**Sevenoaks TN13 3AJ (GB)**

Remarks:
This application was filed on 25-05-2020 as a divisional application to the application mentioned under INID code 62.

## (54) CATION EXCHANGE POLYMERS

(57) The present invention provides novel compositions and methods for removal of protein aggregates from a sample in a flow-through mode.

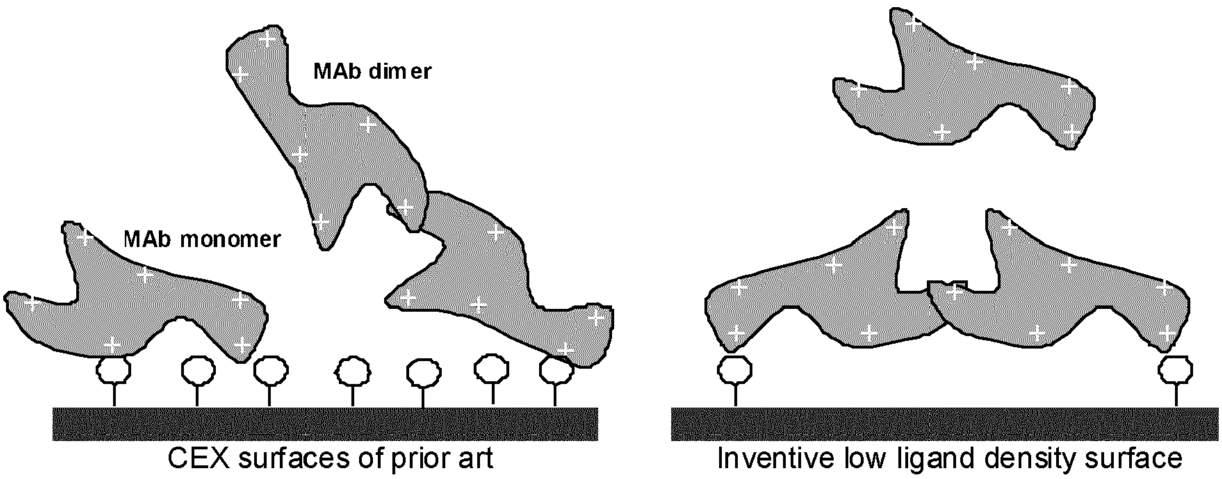


Figure 1

EP 3 730 510 A1

## Description

### Related Applications

The present application claims the benefit of priority of U.S. Provisional Patent Application No. 61/609,533, filing date March 12, 2012, and U.S. Provisional Patent Application No. 61/666,578, filing date June 29, 2012, each of which is incorporated by reference herein in its entirety.

### Field of the Invention

This invention relates to methods of removing protein aggregates from biopharmaceutical preparations containing a product of interest in a flow-through mode.

### Background of the Invention

Protein aggregates are one of the important impurities that need to be removed from biopharmaceutical preparations containing a product of interest, *e.g.,* a therapeutic protein or an antibody molecule. For example, protein aggregates and other contaminants must be removed from biopharmaceutical preparations containing a product of interest before the product can be used in diagnostic, therapeutic or other applications. Further, protein aggregates are also often found in antibody preparations harvested from hybridoma cell lines, and have to be removed prior to the use of the antibody preparation for its intended purpose. This is especially important in case of therapeutic applications and for obtaining Food and Drug Administration approval.

Removal of protein aggregates can be challenging as often there are similarities between the physical and chemical properties of protein aggregates and the product of interest in a biopharmaceutical preparation, which is often a monomeric molecule. There are many different methods in the art for the removal of protein aggregates from biopharmaceutical preparations including, for example, size exclusion chromatography, ion exchange chromatography and hydrophobic interaction chromatography.

Several bind and elute chromatography methods are known for separation of protein aggregates from the product of interest. For example, hydroxyapatite has been used in the chromatographic separation of proteins, nucleic acids, as well as antibodies. In hydroxyapatite chromatography, the column is normally equilibrated, and the sample applied, in a low concentration of phosphate buffer and the adsorbed proteins are then eluted in a concentration gradient of phosphate buffer (see, *e.g.,* Giovannini, Biotechnology and Bioengineering 73:522-529 (2000)). However, in several instances, researchers have been unable to selectively elute antibodies from hydroxyapatite or found that hydroxyapatite chromatography did not result in a sufficiently pure product (see, *e.g.,* Jungbauer, J. Chromatography 476:257-268 (1989); Giovannini, Biotechnology and Bioengineering 73:522-529 (2000)).

Additionally, ceramic hydroxyapatite (CHT), a commercially available chromatography resin, has been used with some success for the removal of protein aggregates, in a resin format (BIORAD CORP, also see, *e.g.,* U.S. patent publication no. WO/2005/044856), however, it is generally expensive and exhibits a low binding capacity for protein aggregates.

A bind and elute cation-exchange chromatography method has also been described, which is sometimes used in the industry for aggregate removal (see, *e.g.* U.S. Patent No. 6,620,918), however it is often observed that an unfavorable trade-off between monomer yield and aggregate removal needs be made. In a recent review of aggregate removal methods from solutions of monoclonal antibodies, it was noted, concerning a bind and elute chromatography mode that "cation exchange chromatography can be a useful way to separate aggregate and monomer but it can be difficult to develop a high yielding step with a high capacity." See, *e.g.,* Aldington et al., J. Chrom. B, 848 (2007) 64-78.

Compared to bind and elute methods and size exclusion chromatography methods known in the art, protein purification in flow-through mode is considered more desirable due to better economics, simplicity, time, and buffer savings.

Attempts have been made in the prior art to implement flow-through aggregate removal based on Hydrophobic Interactions Chromatography (HIC) media (see, *e.g.* U.S Patent No. 7,427,659). However, HIC-based preparative separations have narrow applicability due to generally difficult process development, narrow operating window, and high concentration of salt required in the buffer.

Weak partitioning chromatography (WPC) is another mode of chromatographic operation, in which the product binds weaker than in the case of bind-elute chromatography but stronger than in the case of flow-through chromatography (See, *e.g.* U.S Patent No. 8,067,182); however, WPC also has certain draw back associated with it including, a narrow operating window and lower binding capacity for impurity removal compared to bind and elute methods.

While, some of the flow-through methods described in the prior art have been reported to bind aggregates, the specificity for binding aggregates relative to the product of interest appears to be low. Further, there appear to be no

known methods in the prior art which exhibit a high specificity for binding lower order protein aggregates such as, *e.g.,* dimers, trimers and tetramers.

## Summary of the Invention

The present invention provides novel and improved compositions as well as flow-through methods which use such compositions for separating a product of interest, *e.g.,* a therapeutic antibody or a monomeric protein from protein aggregates in a biopharmaceutical composition. The compositions and methods described herein are especially useful for separating a monomeric protein of interest from lower order protein aggregates, such as, *e.g.,* dimers, trimers and tetramers, which are generally difficult to separate from the monomeric protein.

The present invention is based, at least in part, on increasing selectivity of binding of protein aggregates compared to a product of interest *(i.e.,* monomeric molecule) to a surface in flow-through mode, thereby to separate the protein aggregates from the product of interest. The present invention is able to accomplish this by the unique design of a surface having a certain density of cation exchange binding groups, thereby facilitating a greater number of protein aggregates to bind to the surface, as compared to the monomeric molecules.

In some embodiments according to the present invention, a flow-through chromatography method of separating a monomeric protein of interest from protein aggregates in a sample is provided, where the method comprises contacting the sample with a solid support comprising one or more cation exchange binding groups attached thereto, at a density of about 1 to about 30 mM, where the solid support selectively binds protein aggregates, thereby to separate the monomeric protein of interest from protein aggregates.

In some embodiments, the solid support used in the methods according to the present invention is selected from a chromatographic resin, a membrane, a porous monolith, a woven fabric and a non-woven fabric.

In some embodiments, the solid support comprises a chromatographic resin or a porous bead.

In some embodiments, the solid support is a porous polyvinylether polymeric bead or a porous crosslinked polymethacrylate polymer bead.

In various embodiments, the chromatographic resin or the porous bead comprises a mean particle size of about 50 microns, or between about 10 and about 500 microns, or between about 20 and about 140 microns, or between about 30 and about 70 microns.

In some embodiments, a solid support is selected from a chromatographic resin or porous bead where the mean particle size is between 10 micron to 500 microns, or between 20 and 200 microns, or between 20 and 90 microns. In a particular embodiment the chromatographic resin or porous bead has a mean particle size of about 50 microns. In general, selectivity may improve with decreasing particle size. One skilled in the art would understand that the mean particle size can be adjusted while maintaining some level selectively for binding protein aggregates based on the needs of a specific application or process.

In some embodiments, the protein aggregates are lower order protein aggregates such as, for example, dimers, trimers and tetramers.

In other embodiments, the protein aggregates are higher order protein aggregates such as, for example, pentamers and higher order.

In some embodiments, the cation exchange group used in the methods according to the present invention is selected from the group consisting of a sulfonic group, a sulfate group, a phosphonic group, a phosphoric group, and a carboxylic group.

In some embodiments, the monomeric protein is an antibody. In a particular embodiment, the antibody is a monoclonal antibody. In other embodiments, the monomeric protein is a recombinant protein, *e.g.,* an Fc-fusion protein. In yet other embodiments, the monomeric protein is a non-antibody molecule.

In some embodiments according to the present invention, a flow-through chromatography method of separating a monomeric protein of interest from protein aggregates in a sample is provided, where the method comprising contacting the sample with a solid support comprising one or more cation exchange binding groups attached thereto at a density of about 1 to about 30 mM, where the solid support binds protein aggregates relative to monomers at a selectivity greater than about 10, thereby to separate the protein of interest from protein aggregates.

In other embodiments, a flow-through chromatography method of reducing the concentration of protein aggregates in a sample is provided, the method comprising the steps of: (a) providing a sample comprising a protein of interest and from about 1 to about 20% of protein aggregates; (b) contacting the sample with a solid support comprising one or more cation exchange binding groups attached thereto, at a density of about 1 to about 30 mM; and (c) collecting a flow-through effluent of the sample, where the concentration of protein aggregates in the effluent is reduced by at least 50% relative to the concentration of the aggregates in (a), thereby reducing the concentration of protein aggregates in the sample.

In some embodiments according to the methods of the present invention, the concentration of the protein of interest in the effluent is at least 80% of the concentration of the protein of interest in (a).

In some embodiments, the ionic conductivity of the sample containing aggregates that is contacted with the said solid support is within a range of about 0.5 to about 10 mS/cm.

In some embodiments, a process for purification of a protein of interest (e.g., a monoclonal antibody) described herein, does not require a bind and elute cation-exchange chromatography step. Accordingly, such a process eliminates the need for salt addition to elution solution and use of subsequent dilution steps.

In some embodiments, a process for purification of a protein of interest (*e.g.,* a monoclonal antibody) is provided, where the process does not require an increase in conductivity. Accordingly, such a process does not require dilution after the cation-exchange step in order to reduce conductivity prior to performing the subsequent flow-through anion-exchange step.

Also encompassed by the present invention are polymers comprising cation exchange groups, where the polymers are attached onto a solid support.

In some embodiments, such a polymer comprises the following chemical structure:

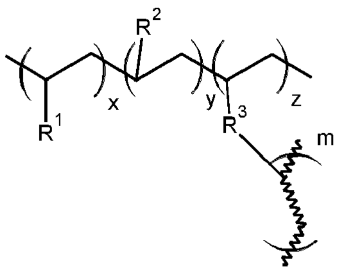

where $R^1$ is a cation-exchange group; $R^2$ is any aliphatic or aromatic organic residue that does not contain a charged group; $R^3$ is any uncharged aliphatic or aromatic organic linker between any two or more polymeric chains; x, y, and z are average molar fractions of each monomer in the polymer, where y>x; and symbol m denotes that a similar polymer chain is attached at the other end of the linker.

In other embodiments, a polymer according to the present invention comprises the following chemical structure:

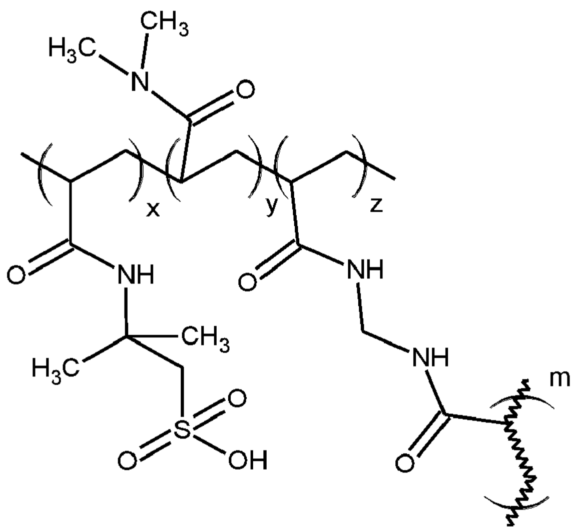

where x, y, and z are average molar fractions of each monomer in the polymer, where y > x; and symbol m denotes that a similar polymer chain is attached at the other end of the linker.

In yet other embodiments, a polymer according to the present invention comprises the following chemical structure, where the polymer is grafted *via* a covalent linkage onto a solid support:

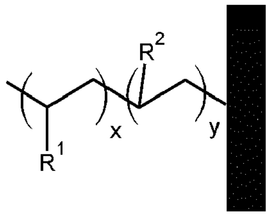

where $R^1$ is a cation-exchange group; $R^2$ is any aliphatic or aromatic organic residue that does not contain a charged group; and x and y are average molar fractions of each monomer in the polymer, where y > x.

In some embodiments, a polymer according to the present invention comprises the following chemical structure:

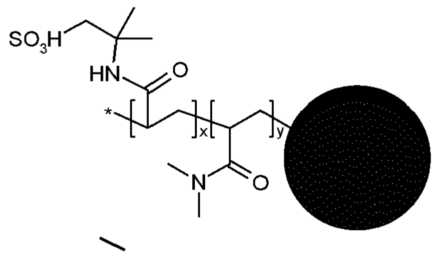

resin

wherein x and y are average molar fractions of each monomer in the polymer, where y > x and wherein the polymer is grafted via linkage onto a chromatography resin.

In various embodiments, polymers are attached to a solid support.

In various embodiments according to the present invention, the effluent containing the product of interest is subjected to one or more separation methods described herein, where the effluent contains less than 20%, or less than 15%, or less than 10%, or less than 5%, or less than 2% protein aggregates.

In some embodiments according to the present invention, the methods and/or compositions of the present invention may be used in combination with one or more of Protein A chromatography, affinity chromatography, hydrophobic interaction chromatography, immobilized metal affinity chromatography, size exclusion chromatography, diafiltration, ultrafiltration, viral removal filtration, anion exchange chromatography, and/or cation exchange chromatography.

In some embodiments, the protein aggregates that are selectively removed by the compositions described herein are higher molecular weight aggregates, i.e. protein pentamers and higher order.

In some embodiments, the protein aggregates that are selectively removed by the compositions described herein comprise lower order aggregate species, such as protein dimers, trimers, and tetramers.

In some embodiments, the solid supports comprising one or more cation exchange binding groups described herein, are used in a flow-through purification process step in a purification process, where the flow-through purification process step as well as the entire purification process may be performed in a continuous manner.

In some embodiments, the solid supports described herein are connected to be in fluid communication with other types of media both upstream and downstream of the solid support. For example, in some embodiments, a solid support comprising one or more cation exchange binding groups, as described herein, is connected to an anion exchange chromatography media upstream and a virus filtration media downstream from the solid support. In a particular embodiment, a sample flows through activated carbon followed by an anion exchange chromatography media followed by a solid support comprising one or more cation exchange binding groups followed by a virus filter. In some embodiments, a static mixer and/or a surge tank is positioned between the anion exchange media and the solid support comprising one or more cation exchange binding groups, in order to perform a pH change.

In all case, the various embodiments can be combined with one another unless technically incompatible or contradictory to one another. For example, embodiments described in relation to particular methods may be used in combination with other processes described herein. Also, the various products and features thereof are useful in the particular methods and products described herein.

## Brief Description of the Drawings

Figure 1 is a schematic depiction of enhanced aggregate selectivity using a composition having a lower density of cation exchange binding groups as compared to a composition known in the art.

Figures 2A-2F depict representative chemical structures of various compositions encompassed by the present invention. Figures 2A-2D depict cross-linked polymeric structures immobilized on a solid support; Figures 2E-2H depict grafted polymeric structures covalently attached to a solid support. $R^1$ is a cation-exchange group such as *e.g.,* sulfonic, sulfate, phosphoric, phosphonic or carboxylic group; $R^2$ is any aliphatic or aromatic organic residue that does not contain a charged group; $R^3$ is any uncharged aliphatic or aromatic organic linker between any two or more polymeric chains; x, y, and z are average molar fractions of each monomer in the polymer, whereas y > x; symbol *m* denotes that a similar polymer chain is attached at the other end of the linker; $R^4$ is NH or O; $R^5$ is a linear or branched aliphatic or aromatic group, such $-CH_2-$, $-C_2H_4-$, $--C_3H_6-$, $-C(CH_3)_2-CH_2-$, -C6H4-; $R^6$ is a linear or branched aliphatic or aromatic uncharged group containing NH, O, or S linker to the polymer chain; and $R^7$ and $R^8$ are independently selected from a group containing one or more neutral aliphatic and aromatic organic residues, and may contain heteroatoms such as O, N, S, P, F, Cl, and the like.

Figure 3A is a graph representing the results of a size exclusion chromatography (SEC) analysis of fractions of a monoclonal antibody (MAb I) passed through three different membrane devices, containing Membrane 7, Membrane 8 or a commercially available membrane (Pall Mustang® S membrane). On the x-axis, the total loading of MAb I on the membrane is shown in g/L and on the y-axis, the relative concentration of MAb I monomer (represented by % yield) compared to the starting concentration is shown.

Figure 3B is a graph representing the results of a size exclusion chromatography (SEC) analysis of fractions of MAb I passed through three membrane devices, containing Membrane 7, Membrane 8 or Pall Mustang® S membrane. On the x-axis, the total loading of MAb I on the membrane is shown in g/L and on the y-axis, the relative concentration of MAb I dimer (represented by % yield) compared to the starting concentration is shown. As observed, the dimer break-through is significantly later for Membrane 7 as compared to both Membrane 8 as well as the Pall Mustang® S membrane.

Figure 3C is a graph representing the results of a size exclusive chromatography (SEC) analysis of fractions of MAb I passed through three membrane devices, containing Membrane 7, Membrane 8 or Pall Mustang® S membrane. On the x-axis, the total loading of MAb I on the membrane is shown in g/L; on the y-axis, the relative concentration of MAb I High Molecular Weight (HMW) aggregate yield compared to the starting concentration is shown. As observed, the HMW break-through is significantly later for Membrane 7 as compared to both Membrane 8 as well as the Pall Mustang® S membrane.

Figure 4A is a graph depicting aggregate breakthroughs, as measured by SEC (shown on the right y-axis), for an antibody pool for Membrane 7 (shown by open triangles) and Membrane 8 (shown by open squares) as a function of MAb loading. Also shown is the monomer (*i.e.,* product of interest) yield in the pool for Membrane 7 (closed triangle) and Membrane 8 (closed square).

Figure 4B is a graph depicting aggregate breakthroughs (shown on the right y-axis) for an antibody pool for Membrane 7 for 2 separate runs (run 1: shown by open circles; run 2: shown by open diamonds) as a function of MAb loading. Also shown is the monomer yield in the pool for Membrane 7 (run 1: shown by closed circles; run 2: shown by closed diamonds).

Figure 5 is a graph depicting aggregate breakthroughs (shown on the right y-axis) for an antibody pool for Membrane 7 as a function of MAb III loading (shown in the x-axis as mg/mL). Also shown is the monomer yield in the pool for Membrane 7 (shown in the left y-axis)

Figure 6A is a graph depicting partition coefficients at pH 5.0 as a function of NaCl concentration for the binding of MAb I monomers to Membrane 8 (open squares), and MAb I aggregates to Membrane 8 (open triangles), MAb I monomers to Membrane 7 (closed squares), and MAb I aggregates to Membrane 7 (closed triangles).

Figure 6B is a graph depicting the partition coefficients at pH 5.0 as a function of NaCl concentration for the binding of MAb II monomers to Membrane 8 (open squares), and MAb II aggregates to Membrane 8 (open triangles), MAb II monomers to Membrane 7 (closed squares), and MAb II aggregates to Membrane 7 (closed triangles).

Figure 7 is a graph representing selectivity plots for the binding of MAb I and MAb II to Membranes 7 and 8, respectively, at pH 5.0. Selectivity of Membrane 8 for MAb I is shown by open squares; selectivity of Membrane 7 for MAb I is shown by closed squares; selectivity of Membrane 8 for MAb II is shown by open triangles; and selectivity of Membrane 7 for MAb II is shown by closed triangles.

Figure 8 depicts a contour plot indicating percentage monomer at 10 and 15 g/L aggregate loadings.

Figure 9 depicts a contour plot indicating optimal region of operation (shown in white) at 5, 10 and 15 g/L aggregate loadings. Optimal was defined as >88% monomer yield and the aggregate making up < 2% of total protein. The regions in grey do not meet these criteria.

Figure 10 is a graph depicting the results of an experiment to investigate effect of flow-rate on throughput of the virus filtration device. The Y-axis denotes pressure drop (psi) and the X-axis denotes throughput of the virus filtration device ($kg/m^2$).

Figure 11 is a schematic depiction of the connected flow-through purification process, which employs the compositions described herein. An activated carbon containing device is connected directly to an anion-exchange device.

The effluent from the anion-exchange device passes through a static mixer, where an aqueous acid is added to reduce pH, and then goes through a cation-exchange flow-through device, according to the present invention, and a virus filter.

Figure 12 is a graph depicting the results of an experiment to measure HCP breakthrough after an anion exchange chromatography media (ChromaSorb™). The X-axis denotes HCP concentration (ppm) and the Y-axis denotes the AEX loading (kg/L)

Figure 13 is a graph depicting the results of an experiment to measure removal of MAb aggregates as a function of loading of the virus filtration device in the flow-through purification process step. The X-axis denotes the virus filtration loading (kg/$m^2$) and the Y-axis denotes percentage of MAb aggregates in the sample after virus filtration.

Figure 14 is a graph depicting the results of an experiment to demonstrate the removal of MAb aggregates as a function of cumulative protein loading, using a cation-exchange resin, as described herein (Lot #12LPDZ119). The X-axis denotes cumulative protein loading in mg/ml, the left Y-axis denotes the concentration of antibody MAb in mg/ml and the right Y-axis denotes the percentage of MAb aggregates in the sample.

Figure 15 is a graph depicting the results of an experiment to demonstrate the removal of MAb aggregates as a function of cumulative protein loading, using a cation-exchange resin, as described herein (Lot # 12LPDZ128). The X-axis denotes cumulative protein loading in mg/ml, the left Y-axis denotes the concentration of antibody MAb in mg/ml and the right Y-axis denotes the percentage of MAb aggregates in the sample.

Figure 16 is a graph depicting the results of an experiment to demonstrate the removal of MAb aggregates as a function of cumulative protein loading, using the a cation-exchange resin, as described herein (Lot # 12LPDZ129). The X-axis denotes cumulative protein loading in mg/ml, the left Y-axis denotes the concentration of antibody MAb in mg/ml and the right Y-axis denotes the percentage of MAb aggregates in the sample.

Figure 17 depicts a chromatogram of resin Lot # 1712 with MAb5 at pH 5 and 3 minutes residence time.

## Detailed Description of the Invention

Several prior art bind and elute as well as flow-through methods have been described in an attempt to separate protein aggregates from monomeric proteins, which are generally the products of interest.

Bind and elute methods are generally time consuming, require significant process development, and sometimes are not successful in effectively separating the aggregates and in particular, lower order aggregates, from a monomeric protein, while maintaining a high yield of monomeric protein. Certain cation-exchange flow-through methods have been described in the art, both with conventional porous resins and membranes; however, they appear to have issues with low capacity of the media for binding aggregates, low selectivity for dimers, and often low yield of the product of interest, *i.e.,* monomeric proteins (see, *e.g.* Liu et al., J. Chrom. A., 1218 (2011), 6943-6952).

Additionally, methods have been described in the prior art which appear to employ cation exchange groups on solid supports to separate proteins. See, *e.g.,* Wu *et* a/. (Effects of stationary phase ligand density on high-performance ion-exchange chromatography of proteins, J. Chrom. 598 (1992), 7-13), which discusses having a high density of cation exchange groups on a solid support in order to have the best chromatographic resolution of two model proteins. However, recently, the effect of cation exchange binding group density on aggregate removal was specifically investigated (see, *e.g.,* Fogle et al., Effects of resin ligand density on yield and impurity clearance in preparative cation exchange chromatography. I. Mechanistic evaluation, J. Chrom. A.. 1225 (2012), 62-69). It was reported that the resolution of monomeric and high molecular weight antibody forms is largely insensitive to the density of binding groups.

WPC has also been described for use of impurity removal (see, *e.g.,* Suda et al., Comparison of agarose and dextran-grafted agarose strong ion exchangers for the separation of protein aggregates, J. Chrom. A, 1216: pp.5256-5264, 2009). In case of weak partitioning chromatography (WPC), the partition coefficient, Kp, ranges from 0.1 to 20; a Kp > 20 is associated with bind-elute chromatography and a Kp<0.1 is associated with flow-through chromatography. WPC has at least two serious drawbacks. First, a narrow operating region (0.1 < Kp < 20), which has to be between flow-through and bind-elute chromatography (see, *e.g.,* U.S. Patent No. 8,067,182). Within this operating region, the selectivity between product and impurities has to be large for efficient separation. Typically for ion-exchange media, the selectivity between product and impurity increases as Kp increases, with highest selectivity under bind-elute conditions. Second, the capacity for impurities is lower than in case of bind-elute mode. Since the Kp values are lower, so will the capacity under typical operating conditions where impurity concentrations are smaller than the that of the product.

The present invention is able to achieve a superior separation of protein aggregates and monomeric proteins, as compared to the various methods described in the art. An important distinction of the present invention from those described in the prior art is the use of a low density of cation exchange groups on a solid support as well as a high specificity for the removal of lower order protein aggregates, *e.g.,* dimers, trimers and tetramers, which are typically more difficult to remove due to their closeness to monomer in size and surface characteristics.

In order that the present invention may be more readily understood, certain terms are first defined. Additional definitions are set forth throughout the detailed description.

## I. Definitions

The term "chromatography," as used herein, refers to any kind of technique which separates the product of interest (*e.g.,* a therapeutic protein or antibody) from contaminants and/or protein aggregates in a biopharmaceutical preparation.

The terms "flow-through process," "flow-through mode," and "flow-through chromatography," as used interchangeably herein, refer to a product separation technique in which a biopharmaceutical preparation containing the product of interest is intended to flow-through a material. In some embodiments, the product of interest flows through the material and the undesirable entities bind to the material. In a particular embodiment, the material contains a certain density of cation exchange binding groups *(i.e.,* lower than the prior art compositions) and is used for separating a monomeric protein from protein aggregates, where the monomeric protein flows through the material, while the protein aggregates bind to the material.

The term "affinity chromatography" refers to a protein separation technique in which a target molecule (e.g., an Fc region containing protein of interest or antibody) specifically binds to a ligand which is specific for the target molecule. Such a ligand is generally referred to as a biospecific ligand. In some embodiments, the biospecific ligand (e.g., Protein A or a functional variant thereof) is covalently attached to a suitable chromatography matrix material and is accessible to the target molecule in solution as the solution contacts the chromatography matrix. The target molecule generally retains its specific binding affinity for the biospecific ligand during the chromatographic steps, while other solutes and/or proteins in the mixture do not bind appreciably or specifically to the ligand. Binding of the target molecule to the immobilized ligand allows contaminating proteins and impurities to be passed through the chromatography matrix while the target molecule remains specifically bound to the immobilized ligand on the solid phase material. The specifically bound target molecule is then removed in its active form from the immobilized ligand under suitable conditions (e.g., low pH, high pH, high salt, competing ligand etc.), and passed through the chromatographic column with the elution buffer, substantially free of the contaminating proteins and impurities that were earlier allowed to pass through the column. It is understood that any suitable ligand may be used for purifying its respective specific binding protein, e.g. antibody. In some embodiments according to the present invention, Protein A is used as a ligand for an Fc region containing target protein. The conditions for elution from the biospecific ligand (e.g., Protein A) of the target molecule (e.g., an Fc- region containing protein) can be readily determined by one of ordinary skill in the art. In some embodiments, Protein G or Protein L or a functional variant thereof may be used as a biospecific ligand. In some embodiments, a process which employs a biospecific ligand such as Protein A, uses a pH range of 5-9 for binding to an Fc-region containing protein, followed by washing or re-equilibrating the biospecific ligand / target molecule conjugate, which is then followed by elution with a buffer having pH about or below 4 which contains at least one salt.

The terms "contaminant," "impurity," and "debris," as used interchangeably herein, refer to any foreign or objectionable molecule, including a biological macromolecule such as a DNA, an RNA, one or more host cell proteins, endotoxins, lipids, protein aggregates and one or more additives which may be present in a sample containing the product of interest that is being separated from one or more of the foreign or objectionable molecules. Additionally, such a contaminant may include any reagent which is used in a step which may occur prior to the separation process. In a particular embodiment, compositions and methods described herein are intended to selectively remove protein aggregates from a sample containing a product of interest.

The term "immunoglobulin," "Ig" or "antibody" (used interchangeably herein) refers to a protein having a basic four-polypeptide chain structure consisting of two heavy and two light chains, said chains being stabilized, for example, by interchain disulfide bonds, which has the ability to specifically bind antigen. The term "single-chain immunoglobulin" or "single-chain antibody" (used interchangeably herein) refers to a protein having a two-polypeptide chain structure consisting of a heavy and a light chain, said chains being stabilized, for example, by interchain peptide linkers, which has the ability to specifically bind antigen. The term "domain" refers to a globular region of a heavy or light chain polypeptide comprising peptide loops (*e.g.,* comprising 3 to 4 peptide loops) stabilized, for example, by β-pleated sheet and/or intrachain disulfide bond. Domains are further referred to herein as "constant" or "variable", based on the relative lack of sequence variation within the domains of various class members in the case of a "constant" domain, or the significant variation within the domains of various class members in the case of a "variable" domain. Antibody or polypeptide "domains" are often referred to interchangeably in the art as antibody or polypeptide "regions". The "constant" domains of antibody light chains are referred to interchangeably as "light chain constant regions", "light chain constant domains", "CL" regions or "CL" domains. The "constant" domains of antibody heavy chains are referred to interchangeably as "heavy chain constant regions", "heavy chain constant domains", "CH" regions or "CH" domains. The "variable" domains of antibody light chains are referred to interchangeably as "light chain variable regions", "light chain variable domains", "VL" regions or "VL" domains. The "variable" domains of antibody heavy chains are referred to interchangeably as "heavy chain variable regions", "heavy chain variable domains", "VH" regions or "VH" domains.

Immunoglobulins or antibodies may be monoclonal or polyclonal and may exist in monomeric or polymeric form, for example, IgM antibodies which exist in pentameric form and/or IgA antibodies which exist in monomeric, dimeric

or multimeric form. The term "fragment" refers to a part or portion of an antibody or antibody chain comprising fewer amino acid residues than an intact or complete antibody or antibody chain. Fragments can be obtained *via* chemical or enzymatic treatment of an intact or complete antibody or antibody chain. Fragments can also be obtained by recombinant means. Exemplary fragments include Fab, Fab', F(ab')2, Fc and/or Fv fragments.

The term "antigen-binding fragment" refers to a polypeptide portion of an immunoglobulin or antibody that binds an antigen or competes with intact antibody (*i.e.,* with the intact antibody from which they were derived) for antigen binding (*i.e.,* specific binding). Binding fragments can be produced by recombinant DNA techniques, or by enzymatic or chemical cleavage of intact immunoglobulins. Binding fragments include Fab, Fab', $F(ab')_2$, Fv, single chains, and single-chain antibodies.

The term "biopharmaceutical preparation," as used herein, refers to any composition containing a product of interest (*e.g.,* a therapeutic protein or an antibody, which is usually a monomer) and unwanted components, such as protein aggregates (*e.g.,* lower order protein aggregates and high molecular weight aggregates of the product of interest).

As used herein, and unless stated otherwise, the term "sample" refers to any composition or mixture that contains a target molecule. Samples may be derived from biological or other sources. Biological sources include eukaryotic and prokaryotic sources, such as plant and animal cells, tissues and organs. The sample may also include diluents, buffers, detergents, and contaminating species, debris and the like that are found mixed with the target molecule. The sample may be "partially purified" (i.e., having been subjected to one or more purification steps, such as filtration steps) or may be obtained directly from a host cell or organism producing the target molecule (e.g., the sample may comprise harvested cell culture fluid). In some embodiments, a sample is a cell culture feed. In some embodiments, a sample which is subjected to the flow-through purification processes described herein is an eluate from a bind and elute chromatography step, e.g., a Protein A affinity chromatography.

The term "protein aggregate" or "protein aggregates," as used interchangeably herein, refers to an association of at least two molecules of a product of interest, *e.g.,* a therapeutic protein or antibody. The association of at least two molecules of a product of interest may arise by any means including, but not limited to, covalent, non-covalent, disulfide, or nonreducible crosslinking.

Aggregate concentration can be measured in a protein sample using Size Exclusion Chromatography (SEC), a well known and widely accepted method in the art (see, *e.g.,* Gabrielson et al., J. Pharm. Sci., 96, (2007), 268-279). Relative concentrations of species of various molecular weights are measured in the effluent using UV absorbance, while the molecular weights of the fractions are determined by performing system calibration following instruction of column manufacturer.

The term "dimer," "dimers," "protein dimer" or "protein dimers," as used interchangeably herein, refers to a lower order fraction of protein aggregates, which is predominantly comprised of aggregates containing two monomeric molecules, but may also contain some amount of trimers and tetramers. This fraction is usually observed as the first resolvable peak in a SEC chromatogram immediately prior to the main monomer peak.

The term "high molecular weight aggregates," or "HMW," as used interchangeably herein, refers to a higher order fraction of protein aggregates, *i.e.* pentamers and above. This fraction is usually observed as one or more peaks in a SEC chromatogram prior to the dimer peak.

The term "binding group," or "ligand" as used interchangeably herein, refers to a specific chemical structure immobilized on a solid support *(e.g.,* a porous surface), which is capable of attracting a monomeric protein or protein aggregates from a solution. Protein attraction to the binding group can be of any type, including ionic, polar, dispersive, hydrophobic, affinity, metal chelating, or van der Waals.

The term "cation exchange binding group," as used herein, refers to a negatively charged binding group. In a particular embodiment, a binding group is a negatively charged sulfonate group.

The term "solid support" refers in general to any material (porous or non porous) to which the binding groups are attached. The attachment of binding groups to the solid support can either be through a covalent bond, such as in the case of grafting, or through coating, adhesion, adsorption, and similar mechanisms. Examples of solid supports used in the methods and compositions described herein include, but are not limited to, membranes, porous beads, winged fibers, monoliths and resins.

The term "density," as used herein, refers to the concentration of binding groups or ligands on a solid support, which is generally expressed as concentration of ligand in moles per liter of porous media. A widely accepted unit of ion-exchange ligand density is milli-equivalent per liter, or meq/L (equivalent to □eq/ml), which corresponds to molar amount of ion-exchangeable groups in a given volume of media. For charged groups with a single ionizable moiety, the ligand density in meq/L would be equivalent to the density of these groups expressed in mmole/L, or mM.

The term "selectivity," as used herein, refers to the dimensionless ratio of partition coefficients of two species between a mobile phase and a stationary phase. A partition coefficient ($K_p$) is the ratio Q/C, where Q and C are the bound and free protein concentrations, respectively.

The term "process step" or "unit operation," as used interchangeably herein, refers to the use of one or more methods or devices to achieve a certain result in a purification process. Examples of process steps or unit operations

which may be employed include, but are not limited to, clarification, bind and elute chromatography, virus inactivation, flow-through purification and formulation. It is understood that each of the process steps or unit operations may employ more than one step or method or device to achieve the intended result of that process step or unit operation. For example, in some embodiments, the clarification step and/or the flow-through purification step may employ more than one step or method or device to achieve that process step or unit operation. In some embodiments, one or more devices which are used to perform a process step or unit operation are single-use devices and can be removed and/or replaced without having to replace any other devices in the process or even having to stop a process run.

As used herein, the term "pool tank" refers to any container, vessel, reservoir, tank or bag, which is generally used between process steps and has a size/volume to enable collection of the entire volume of output from a process step. Pool tanks may be used for holding or storing or manipulating solution conditions of the entire volume of output from a process step. In various embodiments according to the present invention, the processes obviate the need to use one or more pool tanks.

In some embodiments, the processes described herein may use one or more surge tanks.

The term "surge tank" as used herein refers to any container or vessel or bag, which is used between process steps or within a process step (e.g., when a single process step comprises more than one step); where the output from one step flows through the surge tank onto the next step. Accordingly, a surge tank is different from a pool tank, in that it is not intended to hold or collect the entire volume of output from a step; but instead enables continuous flow of output from one step to the next. In some embodiments, the volume of a surge tank used between two process steps or within a process step in a process or system described herein, is no more than 25% of the entire volume of the output from the process step. In another embodiment, the volume of a surge tank is no more than 10% of the entire volume of the output from a process step. In some other embodiments, the volume of a surge tank is less than 35%, or less than 30%, or less than 25%, or less than 20%, or less than 15%, or less than 10% of the entire volume of a cell culture in a bioreactor, which constitutes the starting material from which a target molecule is to be purified.

In some embodiments described herein, a surge tank is used upstream of a step which employs the solid support described herein.

The term "continuous process," as used herein, refers to a process for purifying a target molecule, which includes two or more process steps (or unit operations), such that the output from one process step flows directly into the next process step in the process, without interruption, and where two or more process steps can be performed concurrently for at least a portion of their duration. In other words, in case of a continuous process, as described herein, it is not necessary to complete a process step before the next process step is started, but a portion of the sample is always moving through the process steps. The term "continuous process" also applies to steps within a process step, in which case, during the performance of a process step including multiple steps, the sample flows continuously through the multiple steps that are necessary to perform the process step. One example of such a process step described herein is the flow-through purification step which includes multiple steps that are performed in a continuous manner, e.g., flow-through activated carbon followed by flow-through AEX media followed by flow-through CEX media which utilizes the solid supports described herein followed by flow-through virus filtration.

The term "anion exchange matrix" is used herein to refer to a matrix which is positively charged, e.g. having one or more positively charged ligands, such as quaternary amino groups, attached thereto. Commercially available anion exchange resins include DEAE cellulose, QAE SEPHADEX™ and FAST Q SEPHAROSE™ (GE Healthcare). Other exemplary materials that may be used in the processes and systems described herein are Fractogel® EMD TMAE, Fractogel® EMD TMAE highcap, Eshmuno® Q and Fractogel® EMD DEAE (EMD Millipore).

The term "active carbon" or "activated carbon," as used interchangeably herein, refers to a carbonaceous material which has been subjected to a process to enhance its pore structure. Activated carbons are porous solids with very high surface areas. They can be derived from a variety of sources including coal, wood, coconut husk, nutshells, and peat. Activated carbon can be produced from these materials using physical activation involving heating under a controlled atmosphere or chemical activation using strong acids, bases, or oxidants. The activation processes produce a porous structure with high surface areas that give activated carbon high capacities for impurity removal. Activation processes can be modified to control the acidity of the surface. In some embodiments described herein, activated carbon is used in a flow-through purification step, which typically follows a bind and elute chromatography step or a virus inactivation step which in turn follows the bind and elute chromatography step. In some embodiments, activated carbon is incorporated within a cellulose media, e.g., in a column or some other suitable device.

The term "static mixer" refers to a device for mixing two fluid materials, typically liquids. The device generally consists of mixer elements (non-moving elements) contained in a cylindrical (tube) housing. The overall system design incorporates a method for delivering two streams of fluids into the static mixer. As the streams move through the mixer, the non-moving elements continuously blend the materials. Complete mixing depends on many variables including the properties of the fluids, inner diameter of the tube, number of mixer elements and their design etc. In some embodiments described herein, one or more static mixers are used in the processes described herein. In a particular embodiment, a static mixer is used for achieving the desired solution change after an anion exchange chromatography step and before

contacting a sample with a cation exchange solid support, as described herein.

## II. Exemplary Solid supports

The present invention provides solid supports having a certain density of binding groups or ligands attached thereto, which bind protein aggregates more favorably than the monomeric form of a protein which is usually the product of interest. Without wishing to be bound by theory, it is contemplated that any suitable solid support may be used in case of the present invention. For example, the solid support can be porous or non-porous or it can be continuous, such as in the form of a monolith or membrane. The solid support could also be discontinuous, such as in the form of particles, beads, or fibers. In either case (continuous or discontinuous), the important features of the solid support are that they have a high surface area, mechanical integrity, integrity in aqueous environment, and ability to provide flow distribution to ensure accessibility of the binding groups.

Exemplary continuous porous solid supports include microporous membranes, *i.e.* having a pore sizes between about 0.05 micron and 10 micron. Porous membranes that may be used in the compositions and methods according to the present invention may be classified as symmetric or asymmetric in nature, which refers to the uniformity of the pore sizes across the thickness of the membrane, or, for a hollow fiber, across the microporous wall of the fiber. As used herein, the term "symmetric membrane" refers to a membrane that has substantially uniform pore size across the membrane cross-section. As used herein, the term "asymmetric membrane" refers to a membrane in which the average pore size is not constant across the membrane cross-section. In some embodiments, in case of asymmetric membranes, pore sizes can vary evenly or discontinuously as a function of location throughout the membrane cross-section. In some embodiments, asymmetric membranes can have a ratio of pore sizes on one external surface to pore sizes on the opposite external surface, which ratio is substantially greater than one.

A wide variety of microporous membranes made from a wide variety of materials may be used in the compositions and methods described herein. Examples of such materials include polysaccharides, synthetic and semi-synthetic polymers, metals, metal oxides, ceramics, glass, and combinations thereof.

Exemplary polymers that can be used to manufacture the microporous membranes that may be used in the compositions and methods described herein include, but are not limited to, substituted or unsubstituted polyacrylamides, polystyrenes, polymethacrylamides, polyimides, polyacrylates, polycarbonates, polymethacrylates, polyvinyl hydrophilic polymers, polystyrenes, polysulfones, polyethersulfones, copolymers or styrene and divinylbenzene, aromatic polysulfones, polytetrafluoroethylenes (PTFE), perfluorinated thermoplastic polymers, polyolefins, aromatic polyamides, aliphatic polyamides, ultrahigh molecular weight polyethylenes, polyvinylidene difluoride (PVDF), polyetheretherketones (PEEK), polyesters, and combinations thereof.

Exemplary commercially available microporous membranes are Durapore® and Millipore Express® available from EMD Millipore Corp. (Billerica, MA); Supor® available from Pall Corp. (Port Washington, NY); and Sartopore® and Sartobran® available from Sartorius Stedim Biotech S.A. (Aubagne Cedex, France).

Other exemplary continuous solid supports are monoliths, such as CIM® monolithic materials available from BIA Separations (Villach, Austria).

Exemplary discontinuous solid supports include porous chromatography beads. As will be readily recognized by those skilled in the art, chromatography beads can be manufactured from a great variety of polymeric and inorganic materials, such polysaccharides, acrylates, methacrylates, polystyrenics, vinyl ethers, controlled pore glass, ceramics and the like.

Exemplary commercially available chromatography beads are CPG from EMD Millipore Corp.; Sepharose® from GE Healthcare Life Sciences AB; TOYOPEARL® from Tosoh Bioscience; and POROS® from Life Technologies.

Other exemplary solid supports are woven and non-woven fibrous materials, such as fiber mats and felts, as well as fibers packed into a suitable housing, for example chromatography column, disposable plastic housing, and the like. Exemplary Solid supports also include winged fibers.

## III. Exemplary Binding groups

A great variety of binding groups or ligands can be attached to solid supports and used for effective removal of protein aggregates from a sample, as described herein. In general, the binding group should be capable of attracting and binding to protein aggregates in a solution. Protein attraction to the binding group can be of any type, including ionic (*e.g.,* cationic exchange groups), polar, dispersive, hydrophobic, affinity, metal chelating, or van der Waals.

Exemplary ionic binding groups include, but are not limited to, sulfate, sulfonate, phosphate, phosphonate, carboxylate; primary, secondary, tertiary amine and quaternary ammonium; heterocyclic amines, such as pyridine, pyrimidine, pyridinium, piperazine, and the like.

Polar groups include a wide variety of chemical entities comprising polarized chemical bonds, such C-O, C = O, C-N, C = N, C ≡ N, N-H, O-H, C-F, C-Cl, C-Br, C-S, S-H, S-O, S=O, C-P, P-O, P=O, P-H. Exemplary polar groups

are carbonyl, carboxyl, alcohol, thiol, amide, halide, amine, ester, ether, thioester, and the like.

Hydrophobic binding groups are capable of hydrophobic interactions. Exemplary hydrophobic groups are alkyl, cycloalkyl, haloalkyl, fluoroalkyl, aryl, and the like.

Affinity binding groups are arrangements of several binding functionalities that in concert provide a highly specific interaction with target protein. Exemplary affinity binding groups include Protein A and Protein G and domains and variants thereof.

In some embodiments, a preferred binding group is an ionic group. In a particular embodiment, a binding group is a negatively charged sulfonate group. In general, negatively charged sulfonate groups have several advantages. For example, they exhibit broad applicability to bind positively charged proteins in solution; the chemistry is inexpensive and straightforward with many synthetic manufacturing methods readily available; the interaction between the binding group and proteins is well understood (See, *e.g.,* Stein et al., J. Chrom. B, 848 (2007) 151-158), and the interaction can be easily manipulated by altering solution conditions, and such interaction can be isolated from other interactions.

## IV. Methods of attaching the binding groups to a solid support and controlling the density of binding groups on the solid support

In the compositions and methods described herein, suitable binding groups are attached to a solid support, where the density of the binding groups on the solid support is controlled, such that to provide a greater capacity to bind protein aggregates versus the product of interest.

The compositions and methods described herein are based on a surprising and unexpected discovery that, a lower density of binding groups on a solid support is more effective in the removal of protein aggregates in a flow-through mode, even though the prior art appears to suggest the desirability to have a high density of binding groups. The compositions and methods described herein are especially effective in the removal of lower order protein aggregates such as, *e.g.,* dimers, trimers and tetramers, in a flow-through mode, which are generally more difficult to separate from the monomeric form of proteins, as compared to higher order aggregates such as, *e.g.,* pentamers and higher.

A variety of methods known in the art and those described herein can be used for attaching binding groups to a solid support for use in the methods described herein. In general, the criteria of successful attachment include achievement of the desired binding group density and low rate of detachment of binding groups (*i.e.,* low leaching of binding groups). The binding groups could be attached directly to a solid support, or could be incorporated into a polymeric molecule, which, in turn, can be attached to a solid support. Alternatively, the binding groups can be incorporated into a cross-linked coating applied onto a solid support, with or without forming a chemical bond between the coating and the solid support.

A number of methods are known in the art for attaching the binding groups to a solid support (*see*, for example, Ulbricht, M., Advanced Functional Polymer Membranes, Polymer, 47, 2006, 2217-2262). These methods include, but are not limited to, direct modification of the solid support with binding groups through suitable coupling chemistry; adsorbing, and attaching polymeric molecules. The latter can be accomplished by either grafting "to" (when the polymer is pre-made before reaction with the surface) and grafting "from" (when the polymerization is initiated using the surface groups).

As described herein, the ability to control the density of binding groups on the surface of the solid support is critically important in order to achieve successful separation of protein aggregates and the product of interest. The density of binding groups, expressed in mole/L of porous media, or M, can be conveniently measured using methods known to those skilled in the art. For example, the density of sulfonic acid groups can be measured using Lithium ion exchange analysis. In this analysis, the hydrogens of the sulfonic acid groups are fully exchanged for Lithium ions, rinsed with water, the Lithium ions are subsequently washed off in a concentrated acid solution, and the Lithium concentration in the acid wash solution is measured using Ion Chromatography.

In protein chromatography, a higher density of ligands (binding groups) has usually been desirable since in general it provides a higher binding capacity (see, *e.g.,* Fogle et al., J. Chrom. A, 1225 (2012) 62-69). Typical ligand density for most commercially available cation exchange (CEX) resins is at least 100 milliequivalents/L, or mM for a monovalent ligand. For example, SP Sepharose Fast Flow and CM Sepharose Fast Flow, both available from GE Healthcare, are listed in the product literature to have the concentration of cation-exchange groups 180-250 mM and 90-130 mM, respectively.

A number of methods exist in the art which may be used for controlling the density of binding groups on a solid support. When the binding groups are attached directly onto the solid support, the density can be controlled by the length of reaction, type and concentration of catalyst, concentration of reagent, temperature, and pressure. When surface pretreatment (activation) of the solid support is required, for example by partial oxidation or hydrolysis, the extent of the pretreatment will also control the density of binding groups that will be attached to such a pre-treated surface.

When the binding groups are incorporated in a polymeric structure, which is either adsorbed, adhered, grafted, or coated onto a solid support, the density of the binding groups can be controlled by the composition of that polymeric

structure. One approach to create the polymeric structure with controlled density of the binding groups is copolymerization, *i.e.* polymerization of two or more different monomer types into a single polymeric structure. A binding group can be a part of one of the monomer types used to create the polymeric structure, while other, neutral monomers can be added to reduce the density of the binding groups.

The choice of monomers used in creating a polymer comprising binding groups is dictated by reactivity of the monomers. Reactivities of various monomer types and the effects on the polymer composition are well studied and documented (*see*, for example, Polymer Handbook, 3rd ed., John Wiley & Sons, 1989, p. II). Well-accepted parameters of monomers that predict the composition of the polymer and its structure are the reactivity ratios of the monomers (*see*, for example, Odian, J., Principles of Polymerization, 4th ed., John Wiley & Sons, 2004, p. 466).

A preferred method to attach the binding groups to the solid support is an *in situ* polymerization reaction that incorporates the binding group into a cross-linked coating applied onto the solid support. This method is disclosed in U.S. Patent Nos. 4,944,879 and 4,618,533, as well as published US Patent Publication No. US2009/208784. This method is facile as well as economical. A charged coating can be created by copolymerizing a charged acrylic monomer, for example, 2-acrylamido-2-methylpropanesulfonic acid (AMPS), with a suitable cross-linker, such as N,N'-methylene-bis-acrylamide (MBAM). U.S. Patent Publication No. US2009208784, incorporated by reference in its entirety herein, discloses a microporous membrane modified with a mixture of AMPS and MBAM that can be used for removal of high molecular weight protein aggregates and for increasing the capacity of nanoporous virus filter. However, the aforementioned patent publication does not discuss controlling the ligand density, as described herein, to achieve selective removal of protein aggregates and especially lower order protein aggregates, such as, dimers, trimers, tetramers etc.

A neutral monomer that can be used for reducing the density of charged binding ligands can be selected from a large group of acrylic, methacrylic and acrylamide monomers such as, for example, acrylamide, hydroxypropyl acrylate, hydroxyethyl acrylate, and hydroxyethylmethacrylate. A preferred monomer is dimethylacrylamide (DMAM). The reactivity ratio of AMPS and DMAM monomers ($r_1$ =0.162, $r_2$ = 1.108) (see, *e.g.,* Polymer Handbook, 3rd ed., John Wiley and Sons, 1989, p. 11/156) predicts that a polymer including these monomers would have a tendency for short blocks of poly(DMAM) spaced by individual AMPS units, thereby reducing the density of binding groups. Selecting the ratio of DMAM and AMPS in the reaction solution is therefore an important method to achieve controlled density of binding groups.

A representative chemical structure of a binding group containing polymer, which is coated onto a solid support, is depicted in Figure 2A. In order for the polymer to be coated, it is generally cross-linked to other polymers. In Figure 2A, the polymeric structure is shown in which $R^1$ is any aliphatic or aromatic organic residue containing a cation-exchange group, such as *e.g.,* sulfonic, sulfate, phosphoric, phosphonic or carboxylic group; $R^2$ is any aliphatic or aromatic organic residue that does not contain a charged group; and $R^3$ is any uncharged aliphatic or aromatic organic linker between any two or more polymeric chains.

In the polymeric structure depicted in Figure 2A, y > x, which means that neutral groups (represented by "$R^2$") are present in a greater number than the charged groups (represented by "$R^1$"). Here, the x, y, and z are average molar fractions of each monomer in the polymer, and range independently from about 0.001 to 0.999. The symbol *m* simply denotes that a similar polymer chain is attached at the other end of the cross-linker.

In some embodiments, the polymer containing binding groups is a block copolymer, meaning that it includes a long string or block of one type of monomer (*e.g.,* containing either neutral or charged binding groups) followed by a long string or block of a different type of monomer (*e.g.,* charged if the first block was neutral and neutral if the first block was charged).

In other embodiments, the polymer containing binding groups contains the monomers in a random order.

In yet other embodiments, the polymer containing binding groups is an alternating copolymer, where each monomer is always adjacent to two monomers of a different kind on either side.

In some embodiments, a representative chemical structure of a binding group containing polymer is depicted in Figure 2B, in which $R^4$ is NH or O; $R^5$ is a linear or branched aliphatic or aromatic group, such $-CH_2-$,-C2H4-, --C3H6-, $-C(CH_3)_2-CH_2-$, -C6H4-; and $R^6$ is a linear or branched aliphatic or aromatic uncharged group containing NH, O, or S linker to the polymer chain.

In other embodiments, a representative chemical structure of a binding group containing polymer is depicted in Figure 2C. $R^7$ and $R^8$ are independently selected from a group containing one or more neutral aliphatic and aromatic organic residues, and may contain heteroatoms such as O, N, S, P, F, Cl, and others.

In yet other embodiments, a representative structure of a binding group containing polymer is depicted in Figure 2D.

Another representative chemical structure of a binding group containing polymer, which is grafted to a solid support, is depicted in Figure 2E. The solid support is depicted as a rectangle. In Figure 2E, the polymeric structure is shown in which $R^1$ is any aliphatic or aromatic organic residue containing a cation-exchange group, such as *e.g.,* sulfonic, sulfate, phosphoric, phosphonic or carboxylic group; $R^2$ is any aliphatic or aromatic organic residue that does not contain a charged group. In the polymeric structure depicted in Figure 2A, y>x, which means that neutral groups (represented by "$R^2$") are present in a greater number than the charged groups (represented by "$R^1$").

In some embodiments, the graft polymer containing binding groups is a block copolymer, meaning that it includes a long string or block of one type of monomer (*e.g.,* containing either neutral or charged binding groups) following by a long string or block of a different type of monomer (*e.g.,* charged if the first block was neutral and neutral if the first block was charged).

In other embodiments, the polymer containing binding groups contains the monomers in a random order.

In other embodiments, the polymer containing binding groups is an alternating copolymer, whereas each monomer is always adjacent to two monomers of a different kind.

In some embodiments, a representative chemical structure of a binding group containing polymer is depicted in Figure 2F, in which $R^4$ is NH or O; $R^5$ is a linear or branched aliphatic or aromatic group, such $-CH_2-$, -C2H4-, -C3H6-, $-C(CH_3)_2-CH_2-$, -C6H4-; and $R^6$ is a linear or branched aliphatic or aromatic uncharged group containing NH, O, or S linker to the polymer chain.

In other embodiments, a representative chemical structure of a binding group containing polymer is depicted in Figure 2G. $R^7$ and $R^8$ are independently selected from a group containing one or more neutral aliphatic and aromatic organic residues, and may contain heteroatoms such as O, N, S, P, F, Cl, and others.

In yet other embodiments, a representative structure of a binding group containing polymer is depicted in Figure 2H.

The sulfonic acid group in Figures 2B-2D and 2F-2H can be in the protonated form as depicted, as well as in the salt form, containing a suitable counterion such as sodium, potassium, ammonium, and the like.

## IV. Devices incorporating the compositions described herein

In some embodiments, solid supports having binding groups attached thereto, as described herein, are incorporated into devices. Suitable devices for solid supports, such as microporous membranes, include filtration cartridges, capsules, and pods. Exemplary devices also include stacked-plate filtration cartridges disclosed in the U.S. Publication Nos. US20100288690 A1 and US20080257814 A1, incorporated by reference herein. In case of these devices, a solid support is permanently bonded to the polymeric housing and the devices have a liquid inlet, an outlet, and a vent opening, and further minimize the volume of retained liquid. Other exemplary devices include pleated filter cartridges and spiral-wound filter cartridges. Yet other exemplary devices are chromatography columns. Chromatography columns can be produced from a number of suitable materials, such as glass, metal, ceramic, and plastic. These columns can be packed with solid support by the end user, or can also be pre-packed by a manufacturer and shipped to the end user in a packed state.

## V. Methods of using the compositions and devices described herein

The devices containing solid supports having binding groups attached thereto *(e.g.,* cation exchange binding groups) can be used for removal of protein aggregates in a flow-through mode. Prior to application for preparative scale separation, the process must be developed and validated for proper solution conditions such as pH and conductivity, and the range of protein loading on the device must be determined. The methods for process development and validation are widely known and routinely practiced in the industry. They usually involve Design of Experiments (DoE) approaches that are illustrated in the Examples herein.

The devices are commonly flushed, sanitized, and equilibrated with an appropriate buffer solution prior to use. Protein solution is adjusted to a desirable conductivity and pH and is subsequently pumped through a device at either constant pressure or constant flow. The effluent is collected and analyzed for the protein yield and aggregate concentration.

In some embodiments, a device for aggregate removal, as described herein, is connected directly to a virus filtration device that is designed to ensure size-based removal of viral particles, for example, as taught in US. Patent No. 7,118,675, incorporated by reference herein in its entirety.

The flow-through aggregate removal step using the compositions and devices described herein can be placed anywhere in a protein purification process, *e.g.,* in an antibody purification process. Table 1 depicts examples of protein purification processes that incorporate flow-through aggregate removal as one or more intermediate steps, which is highlighted in bold. It is understood that many variations of these processes may be used.

"Protein capture" step, as described herein, refers to the step in a protein purification process which involves isolating the protein of interest from the clarified or unclarified cell culture fluid sample by performing at least the following two steps: i) subjecting the cell culture fluid to a step selected from one or more of: adsorption of the protein of interest on a chromatography resin, a membrane, a monolith, a woven or non-woven media; precipitation, flocculation, crystallization, binding to a soluble small molecule or a polymeric ligand, thereby to obtain a protein phase comprising the protein of interest such as, *e.g.,* an antibody; and (ii) reconstituting the protein of interest by eluting or dissolution of the protein into a suitable buffer solution.

Bind/elute purification is an optional process step consisting of binding the protein of interest to a suitable chromatography media, optionally washing the bound protein, and eluting it with appropriate buffer solution.

Flow-through AEX polishing is an optional process step consisting of flowing the solution of protein of interest through a suitable AEX chromatography media without significantly binding of the protein of interest to the media.

Activated Carbon Flow-through is an optional purification step designed to remove various process-related impurities, as described in co-pending provisional patent application no. 61/575,349, incorporated by reference herein.

Virus filtration consists of flowing the protein solution through a porous membrane, which can be in the form of flat sheet or hollow fiber that retains the viral particles to high degree of LRV, while passing substantially all protein of interest.

Table 1

| | Step 1 | Step 2 | Step 3 | Step 4 | Step 5 |
|---|---|---|---|---|---|
| Process A | Antibody capture | Bind/elute Purification | Flow-through AEX polishing | Flow-through aggregate removal | Virus Filtration |
| Process B | Antibody capture | Flow-through aggregate removal | Bind/elute Purification | Flow-through AEX polishing | Virus Filtration |
| Process C | Antibody capture | Bind/elute Purification | Flow-through aggregate removal | Flow-through AEX polishing | Virus Filtration |
| Process D | Flow-through aggregate removal | Antibody capture | Bind/elute Purification | Flow-through AEX polishing | Virus Filtration |
| Process E | Antibody capture | Flow-through aggregate removal | Flow-through AEX polishing | Virus Filtration | |
| Process F | Antibody capture | Flow-through aggregate removal | Flow-through AEX polishing | Bind/elute Purification | Virus Filtration |
| Process G | Antibody capture | Flow-through AEX polishing | Flow-through aggregate removal | Bind/elute Purification | Virus Filtration |
| Process H | Antibody capture | Flow-through AEX polishing | Flow-through aggregate removal | Virus Filtration | |
| Process I | Antibody capture | Activated Carbon Flow-through | Flow-through AEX polishing | Flow-through aggregate removal | Virus Filtration |

It is understood that in the Table 1 above, the step of Antibody Capture, as well as Bind/Elute Purification, can be operated in any of three modes: (1) batch mode, where the capture media is loaded with target protein, loading is stopped, media is washed and eluted, and the pool is collected; (2) semi-continuous mode, wherein, the loading is performed continuously and the elution is intermittent, e.g., in case of a continuous multicolumn chromatography procedure employing two, three, or more columns; and (3) full continuous mode, where both loading and elution are performed continuously.

The optimal flow rate used with the flow-through cation exchange solid support described herein can sometimes have an effect on the aggregate removal performance of the solid support and, when the solid support is positioned upstream of a virus filter, it can also affect the performance of the virus filter. The optimal flow rate can be readily determined in a simple set of experiments using the protein solution of interest. Typical flow rates fall in the range between about 0.05 and 10 CV/min.

Some exemplary processes described in Table 1, in particular Process E, Process H, and Process I, do not include a bind and elute cation-exchange chromatography step, while still ensuring aggregate removal using the methods described herein. Elimination of the bind and elute cation-exchange chromatography step offers a number of significant advantages for the downstream purification process, *i.e.* savings of process time, simplification of process development, elimination of cleaning and cleaning validation, *etc.* Another strong advantage is the elimination of high-conductivity elution, allowing for the entire downstream process to be performed without addition of salt and then without subsequent dilutions.

This invention is further illustrated by the following examples which should not be construed as limiting. The contents of all references, patents and published patent applications cited throughout this application, as well as the Figures, are incorporated herein by reference.

## Examples

### Example 1. Preparation of cation-exchange (CEX) surface-modified membrane

In this experiment, a series of CEX surface-modified membranes were prepared with a variable density of binding groups, which in this case are negatively charged sulfonic acid residues. The density of the cation exchange groups was controlled by formulation of reactive solution used for surface modification. In order to achieve a lower density, an uncharged reactive monomer, N,N-dimethylacrylamide, was added in different amounts.

A series of aqueous solutions were prepared containing 2-acrylamido-2-methylpropanesulfonic acid (AMPS) ranging from 0 to 4.8%wt., N,N-dimethylacrylamide ranging from 0 to 4.8%wt., and 0.8% wt. of N, N'-methylenebisacrylamide. A hydrophilic ultra-high molecular weight polyethylene membrane with pore size rating of 0.65 um and a thickness of 0.125 mm was cut into square pieces of 14 cm by 14 cm and each piece was submerged in one of solutions for 30 seconds to ensure complete wetting. The excess solution was nipped off, and the membrane was exposed to 2 MRads of electron beam radiation under inert atmosphere. The membrane was subsequently rinsed with deionized water and dried in air.

Table 2 lists the variants of CEX surface modified membranes that were prepared. It was determined using Lithium ion exchange that the anionic group density was 0.13 mmol/g for Membrane 8 (no DMAM added) and 0.08 mmol/g for Membrane 7 (1:1 AMPS:DMAM by weight), which correspond to ligand densities of 34 and 21 mM, respectively.

### Example 2. Analysis of aggregate binding selectivity using static capacity measurements

In this experiment, CEX membranes with varied density of binding groups prepared in Example 1, were tested for their selectivity for binding protein monomers and protein aggregates.

A 2 g/L solution of a partially purified monoclonal antibody, referred to as MAb I, containing about 15% aggregates was prepared in 50 mM Sodium Acetate buffer, pH 5.0. A 14 mm membrane disk was pre-soaked in the acetate buffer and then transferred to 0.5 mL of antibody solution. The solution vials were gently shaken for 15 hours, and the molecular weight species left in solution were analyzed by Size-Exclusion Chromatography. The results are presented in Table 2.

While the generally low yields of MAb indicates that the membranes have been loaded at below capacity in this experiment, one of the variants, Membrane 7, demonstrated practically complete removal of dimers and high molecular weight (HMW) aggregates. This indicates that strong aggregate binding selectivity can be achieved.

Table 2.

| **Membrane Sample No.** | **AMPS wt.% (CEX binding group)** | **DMAM wt. % (neutral group)** | **MAb monomer left in solution yield (%)** | **MAb dimer left in solution yield (%)** | **MAb HMW aggregate left in solution yield (%)** |
|---|---|---|---|---|---|
| 1 | 0 | 4.8 | 82.5 | 86.1 | 80.4 |
| 2 | 0.1 | 4.7 | 80.8 | 69.2 | 81.8 |
| 3 | 0.2 | 4.6 | 86.6 | 75.4 | 94.5 |
| 4 | 0.4 | 4.4 | 88.4 | 77.9 | 106.5 |
| 5 | 0.6 | 4.2 | 78.0 | 63.5 | 53.7 |
| 6 | 1 | 3.8 | 70.3 | 32.3 | n/d |
| 7 | 2.4 | 2.4 | 29.4 | 1.6 | n/d |
| 8 (prior art membrane described in WO 2010098867) | 4.8 | 0 | 44.4 | 14.7 | n/d |
| 9 | Unmodified membrane control | | 80.4 | 69.0 | 74.7 |
| n/d - not detected | | | | | |

**Example 3. Removal of aggregates in flow-through mode for a partially purified monoclonal antibody (MAbl)**

In a representative experiment, successful use of the membranes according to the present invention for the removal of protein aggregates from a sample containing a monoclonal antibody in flow-through mode, was demonstrated.

Five layers of membrane 7 from Example 1 were sealed into a vented polypropylene device, with a frontal membrane filtration area of 3.1 $cm^2$ and membrane volume of 0.2 mL. This type of device is referred to below as the "Micro" device. A purified MAb I at 4.5g/L was dialyzed into pH 5.0, 50 mM acetate buffer. The resulting material, referred to as partially purified MAb I, was diluted to a concentration of 1 g/L Mab I with 5 % aggregates, in pH 5, 50 mM acetate with a conductivity of ~3.0 mS/cm. The material (about 80-100 mL) was then passed through 0.2 mL devices containing either membrane 7 or 8 from Example 1, or a 0.18 mL Acrodisk device containing Pall Mustang® S membrane (commercially available from Thermo Fisher Scientific, Inc., Waltham, MA) at ~1 CV/min. Prior to passing the MAb, the membrane devices were wetted with 18 mΩ water and equilibrated with 50 column volumes (CV) of 50 mM sodium acetate, pH 5.0. The flow-through of the MAb solution was collected for analysis using analytical size exclusion chromatography (SEC). The results are shown in Figures 3A-3C. It is clear that Membrane 7 with a lower density of AMPS binding groups compared to Membrane 8, as established in Example 1, offers a superior selectivity for binding aggregates.

Table 3 summarizes aggregate binding capacity of the three membranes measured at about 20% aggregate breakthrough. As demonstrated, Membrane 7 exhibits almost a 3-fold increase in aggregate binding capacity as compared to commercially available membranes, *e.g.,* the Pall Mustang® S membrane, as well as Membrane 8.

Table 3. Device loading and capacity at dimer breakthrough around 20%

| Membrane | At%Dimer Breakthrough | Membrane Loading (g/L) | Dimercapacity (mq/mL) | Monomer yield (%) |
|---|---|---|---|---|
| 7 | 16.2 | 300 | 10.7 | 89.2 |
| 8 | 23.1 | 120 | 3.9 | 77.1 |
| PALL Mustang S | 28.6 | 120 | 3.7 | 71.0 |

**Example 4. Removal of aggregates in flow-through mode for a partially purified monoclonal antibody (MAb II)**

In another experiment, successful use of membranes according to the present invention in the removal of protein aggregates from a sample containing a different monoclonal antibody, is demonstrated.

Protein A purified MAb II was adjusted to pH 5.0 using 1 M Tris base, and to a conductivity of 3.4 mS/cm using 5 M NaCl solution. The resulting material referred to partially purified MAb II had a concentration of 6 g/L with 2.4 % aggregates. The material (about 18 mL) was then passed through 0.2 mL devices containing either membrane 7 or 8 from Example 1. Prior to passing the MAb through the membrane, the membrane was wetted with 18 mΩ water and equilibrated with 50 column volumes (CV) of 50 mM sodium acetate, pH 5.0. The flow-through of the MAb solution was collected for analysis using analytical size exclusion chromatography (SEC). The first 2 fractions were ~4.5 mL each, while the rest of the 10 fractions were 0.85 mL each. After the MAb treatment, the membrane was washed with 20 CVs of 50 mM sodium acetate, pH 5.0. The bound protein was eluted using 50 mM sodium acetate, pH 5.0 + 1 M NaCl in 30 CVs. The total amount of MAb II loaded on the membranes was 531 mg/ml. The MAb monomer yield was calculated based on amount of monomer in the flow-through versus total monomer passed through the membrane.

As shown in Figure 4A, very little aggregate (<0.1% in the pool) was seen in the breakthrough pool of Membrane 7 up to ~ 13 g/L aggregate-loading while aggregate breakthrough was observed for Membrane 8 even in the first fraction collected indicating superior aggregate capacity for Membrane 7. The amount of aggregates in the breakthrough pool for Membrane 8 was 0.8 %. At an aggregate-loading of 13 g/L, the monomer yields were 93 and 86 for Membrane 8 and Membrane 7, respectively. Although the yield was slightly lower, no aggregate breakthrough was observed for Membrane 7. A later breakthrough of the aggregates is an indication of higher aggregate binding capacity. These breakthrough curves can be used to guide the design of a preparative scale aggregate removal process or device. For example, a device containing Membrane 7 can be loaded to at least 500 g/L to achieve pool purity of < 0.1 % aggregates. Generally, higher loading is very desirable in order to increase the monomer yield and reduce the overall cost associated with the process. In addition, higher yield can be achieved by increasing the loading until aggregate breakthrough is observed or modifying the solution conditions. The flow-through data clearly demonstrates the superior performance of Membrane 7 for the removal of aggregates.

Figure 4B shows monomer yield and aggregate levels in the breakthrough pools for two different runs using two different lots of Membrane 7. Run 2 was loaded to 700 mg/mL compared to run 1 at 530 mg/ml. The solution

conditions (pH and conductivity) were similar in both the runs while the MAb concentrations were different (run 1: 6 g/L; run 2: 4.4 g/L). The amount of aggregates in the pool was similar for both the runs, while there were some differences in the yield (however, it is anticipated that the differences in yields might be attributed to general variability of analytical techniques used for measuring MAb concentrations). Interestingly, even at a MAb loading of 700 g/mL the amount of aggregates in the pool is only 0.25% (run2; Figure 4B) which indicates a binding capacity for aggregates to be > 15 g/L.

**Example 5. Removal of aggregates in flow-through mode for a partially Purified monoclonal antibody (MAb III) containing high pH-induced aggregates**

In another experiment, successful use of membranes according to the present invention in the removal of protein aggregates from a sample containing a yet another monoclonal antibody, MAbIII, is demonstrated.

A solution of partially purified monoclonal IgG feed (MAb III) was prepared to 6 g/L in pH 5, 50 mM acetate buffer with a conductivity of 3.5 mS/cm. The MAb III had been previously shocked to high pH (11) in order to generate 3.7% total aggregates (measured by SEC-HPLC). A Micro filtration device with a filtration area of 3.1 cm2 and a volume of 0.2 mL was pre-molded using 5 layers of Membrane 7. Prior to passing the MAb, the membrane was wetted with 18 mΩ water and equilibrated with 50 column volumes (CV) of 50 mM sodium acetate, pH 5.0. The flow-through of the MAb solution was at 2.5 CV/min and fractions were collected for analysis using analytical size exclusion chromatography (SEC). The total amount of MAb III loaded onto the membranes was 930 mg/ml. The monomer yield was calculated based on amount of monomer in the flow-through versus total monomer passed through the membrane.

Similar to Examples 3 and 4 for MAb I and II, Membrane 7 showed a high selectivity and high binding capacity (> 10 mg/mL) for MAb III aggregates, as shown in Figure 5.

**Example 6: Purification of protein monomers and protein aggregates**

In this experiment, preparations of pure antibody monomers and aggregates were generated using preparative SEC for further characterization of Membrane 7.

Monomers and aggregates were purified from a mixture using the Sephacryl S-300 HR (GE Healthcare) resin. 4.5 mL of the partially purified MAb at 4.5 - 6 g/L was passed through a 330 mL column. Prior to MAb injection, the column was equilibrated with 1 column volume (CV) of PBS. Two elution fractions were collected: aggregate and monomer fractions. The aggregate fraction was concentrated 10X using 3000 MW cut-off Amicon UF membrane and analyzed using analytical SEC. The aggregate content was >70% in these fractions with a concentration of ~0.5 g/L.

**Example 7: Determination of steric mass action (SMA) model parameters: characteristic charge (v) and SMA equilibrium constant ($K_{SMA}$).**

The following experiment was designed to elucidate the mechanism of aggregate binding selectivity observed for the membranes according to the present invention. Using the Steric Mass Action (SMA) model (see, *e.g.,* Brooks, C. A. and Cramer, S. M., Steric mass-action ion exchange: Displacement profiles and induced salt gradients. AlChE Journal, 38: pp. 1969-1978, 1992), the number of aggregate interactions with the surface (v) and the affinity constant ($K_{SMA}$) for the monomer and aggregates were measured, to reflect the affinity of the monomers and the aggregates for the surface.

The SMA model has been used successfully to explain some of the complexities of nonlinear ion-exchange interactions. The model takes into account the sites that are not available to protein molecules by considering both the sites that are "lost" (e.g., or not available for binding) due to direct protein-ligand interaction and sites that are sterically "lost" due to protein hindrance. The quantity of sites "lost" due to steric hindrance is assumed to be proportional to the bound protein concentration. The SMA equation for a single component system is given by:

$$C = \frac{Q}{K_{SMA}}\left(\frac{C_{salt}}{Q_{salt}}\right)^{\nu} = \frac{Q}{K_{SMA}}\left(\frac{C_{salt}}{\Lambda - (\sigma + \nu)Q}\right)^{\nu}$$

(1)

where Q and C are the bound and free protein concentrations, respectively. K is the SMA equilibrium association constant; $\nu$ is the characteristic charge; $\sigma$ is the steric factor; $C_{salt}$ is the free salt concentration; $Q_{salt}$ is the bound salt concentration available for ion-exchange; and $\Lambda$ is the total ionic capacity of the resin. Characteristic charge is the average number of sites on the modified solid support occupied by a protein through ionic interactions, and steric factor is the number of

sites sterically hindered by a protein upon binding to the adsorbent.

For linear region of the adsorption isotherm (when Q is very small), Λ> > (σ+ v)Q, and we can approximate the above equation to:

$$\frac{Q}{C} = K_p = K_{SMA}\left(\frac{\Lambda}{C_{salt}}\right)^{\nu}$$

(2)

The term Q/C is defined as the partition coefficient ($K_p$), and ratio of partition coefficients is defined as selectivity (S). An alternative method of determining the partition coefficient is by calculating the slope of the adsorption isotherm in the linear region (*e.g.,* as described in U.S. Patent No. 8,067,182).

In one experiment, 0.2 mL membrane Micro devices (containing either Membrane 7 or 8) were wetted using water, and equilibrated with 20 CV (4 mL) of 50 mM sodium acetate, pH 5.0 (buffer A). 100 μl of purified monomer or aggregate (as per the purification process described in Example) at 0.5 g/L was injected. The bound protein was eluted using a 20 CV gradient of buffer A and buffer A + 500 mM NaCl. The salt concentration for the isocratic runs was chosen based on the salt concentration (conductivity) range in which the protein elutes. The isocratic run was performed by loading 100μl of protein onto the membrane. The running buffer was buffer A + salt (different salt concentrations based on protein elution range were used as described above). The elution chromatogram was monitored using UV280 or UV230 signal, and the retention volume at the protein peak was noted. The SMA parameters were determined using the isocratic elution method as described in Pedersen, et al, (Pedersen, et. al., Whey proteins as a model system for chromatographic separation of proteins, J. Chromatogr. B, 790: pp. 161-173, 2003).

Table 4 summarizes the SMA parameters for MAb I for Membranes 8 and 7. Table 4 summarizes the SMA parameters for MAb II for Membranes 8 and 7. $K_{SMA}$ was determined assuming an ionic capacity of 34 and 21 mM for Membrane 8 and 7, respectively. Membrane porosity of 78% was used.

Table 4. $S_{MA}$ parameters for MAb I

| MAb I | Species | V | $K_{SMA}$ |
|---|---|---|---|
| Membrane 8 | Monomer | 13.2 | 1.04E13 |
| | Aggregates | 13.55 | 1.00E13 |
| Membrane 7 | Monomer | 9.66 | 4.21E10 |
| | Aggregates | 14.0 | 2.32E14 |

Table 5. SMA parameters for MAb II

| MAb II | Species | V | $K_{SMA}$ |
|---|---|---|---|
| Membrane 8 | Monomer | 9.8 | 2.79E9 |
| | Aggregates | 11.2 | 4.44E10 |
| Membrane 7 | Monomer | 13.4 | 2.11E12 |
| | Aggregates | 23.4 | 9.06E21 |

For both MAbs, it was observed that the difference in the characteristic charge of monomer and aggregates is higher for Membrane 7 indicating a greater number of interactions for aggregates than monomers. In addition, the affinity (as seen from $K_{SMA}$) is higher for Membrane 7 than Membrane 8.

As depicted in Figures 6A and 6B, it was observed that the partition coefficient (a measure of affinity) for aggregates is higher than that observed for monomers in case of both Membranes 8 and 7, thereby indicating stronger binding to aggregates than monomers. It was also observed that the effect is more pronounced in case of Membrane 7 relative to Membrane 8, demonstrating a superior performance of the former for aggregate removal.

As depicted in Figure 7, Membrane 7 has a higher selectivity (S = ratio of Kp of aggregate to Kp of monomer) than Membrane 8 for two different MAbs (MAb I and MAB II) at pH 5.0 at all salt concentrations. Notably, the selectivity

increases as the salt concentration decreases for both membranes. And, interestingly the increase in selectivity is much higher for Membrane 7 than Membrane 8, indicating that higher selectivity could be realized even at lower density of the binding groups. Also, as depicted in Figure 7, the maximum performance benefits (higher monomer yield and higher aggregate removal) were realized even at lower salt concentrations (or higher selectivity).

**Example 8: Determination of operating window for Membrane 7**

In a representative experiment, it was demonstrated using a Design of Experiments approach (DoE), that a practical process window, *i.e.* combination of solution pH, ion conductivity, and protein loading on the membranes according to the present invention, can be achieved. A Design of Experiments (DoE) approach is a widely accepted engineering tool for identifying reliable operating conditions (*see*, for example, Anderson, M.J. and Whitcomb, P.J. 2010 Design of Experiments. Kirk-Othmer Encyclopedia of Chemical Technology. 1-22).

A central composite surface response using the DoE approach with 3 factors was performed in batch mode for Membrane 7 in order to determine its operating window. The parameters that were investigated were: pH, conductivity and aggregate loading. Various amounts of partially purified MAb I (0.5 - 2.1 mL) at various conditions (Table 5) were incubated with 13$\mu$l of membrane for 20 hrs. The supernatant was then analyzed for aggregates and yield using analytical SEC.

Table 6. Runs and results of a 3 factor, central composite surface response Design of experiments approach

| Label | pH | Cond (mS/cm) | Agg. load (g/L) | % monomer | % yield |
|---|---|---|---|---|---|
| 1 | 4 | 3 | 5 | 100 | 78.4 |
| 2 | 6 | 3 | 5 | 100 | 85.9 |
| 3 | 4 | 10 | 5 | 100 | 77.7 |
| 4 | 6 | 10 | 5 | 95.6 | 100 |
| 5 | 4 | 3 | 15 | 98.1 | 92.8 |
| 6 | 6 | 3 | 15 | 98.0 | 95.3 |
| 7 | 4 | 10 | 15 | 98.1 | 92.6 |
| 8 | 6 | 10 | 15 | 96.0 | 100 |
| 9 | 4 | 6.5 | 10 | 98.7 | 86.5 |
| 10 | 6 | 6.5 | 10 | 95.9 | 99.2 |
| 11 | 5 | 3 | 10 | 100 | 86.5 |
| 12 | 5 | 10 | 10 | 96.7 | 98.1 |
| 13 | 5 | 6.5 | 5 | 100 | 77.8 |
| 14 | 5 | 6.5 | 15 | 97.8 | 92.6 |
| 15 | 5 | 6.5 | 10 | 98.5 | 88.9 |
| 16 | 5 | 6.5 | 10 | 98.4 | 88.9 |
| 17 | 5 | 6.5 | 10 | 98.5 | 88.9 |
| 18 | 5 | 6.5 | 10 | 98.6 | 88.9 |
| 19 | 5 | 6.5 | 10 | 98.0 | 88.9 |
| 20 | 5 | 6.5 | 10 | 98.0 | 88.9 |

This DoE study highlighted three key operating parameters - loading, pH, and conductivity in determining membrane performance for yield and aggregate removal and suggested a simple procedure to define the desired operating window. The results are plotted in Figures 8 and 9.

**Example 9. Use of membranes according to the present invention in the protection of downstream Virus filter during antibody purification using a feed stream containing heat-induced aggregates**

This example demonstrates that the membranes comprising one or more cation exchange groups described herein can be successfully used to increase the throughput of a downstream virus filter in a purification process.

In general, it has been previously reported that a surface-modified membrane to pre-treat the antibody feed can be used before virus filtration (see, for example, U.S. Patent No. 7,118,675 and PCT publication no. WO 2010098867, incorporated by reference herein). Because of the high cost of virus filtration, increasing the filter throughput has a direct effect on the final cost of the protein product. A number of commercial products are currently marketed specifically to increase the throughput of virus filters, including those available from EMD Millipore Corporation, *e.g.,* Viresolve® Prefilter and Viresolve® Pro Shield. However, as demonstrated herein, the membranes according to the present invention are far superior in protecting downstream virus filters, as compared to the those described in the prior art or presently commercially available.

A Heat-Shocked polyclonal IgG feed for testing the protection of a virus filter was prepared at 0.1 g/L, using the IgG from SeraCare Life Sciences (Milford, MA), Human Gamma Globulin 5% Solution, Catalog # HS-475-1L, in either pH 5, 50 mM acetate at 8.1 or 16.0 mS/cm (using NaCl) by the following procedure. One liter of the 0.1 g/L solution (at 8.1 or 16.0 mS/cm) was stirred at 170 rpm while heated in a constant water bath set to 65° Celsius for 1 hour after reaching temperature. The solution was removed from the heat and stirring and allowed to cool to room temperature for 3 hours and then refrigerated at 4°C overnight. The next day the Heat-Shocked polyclonal IgG was allowed to warm to room temperature. Fresh solutions of 0.1 g/L polyclonal IgG were prepared in the appropriate sterile filtered pH and conductivity buffers (8.1 or 16.0 mS/cm).

The final solutions for throughput testing were prepared using 9% by volume of the heat-shocked polyclonal IgG stock solutions and the freshly prepared 0.1 g/L polyclonal IgG solutions. Final pH and conductivity adjustments were made using 10 M HCl, NaOH, or 4 M NaCl. Micro filtration devices with a filtration area of 3.1 $cm^2$ were pre-molded using 3 layers of membrane and put in series at a 1:1 area ratio with Viresolve® Pro devices (EMD Millipore Corp., Billerica, MA). Both devices were prewetted and vented to remove air using the buffer only, pH 5, 50 mM acetate, 8.1 or 16.0 mS/cm. Under constant pressure of 30 psi, an initial flux in mL of buffer/min throughput was measured by mass over a 15 minute period to determine an initial constant flux value. The feed was switched to the heat-shocked polyclonal IgG feed at 30 psi and the volume throughput was measured and plotted versus time until the flux decayed to 25% the initial buffer only flux. The total throughput of heat-shocked polyclonal IgG was measured in L/$m^2$ at V75 and converted to kg of polyclonal IgG/$m^2$ membrane. As can be seen from Table 6, the Membrane 7 provided superior protection to a virus removal filter (greater volumetric throughput) than Membrane 8.

Table 7

| | V75 throughput (L/$m^2$) | |
|---|---|---|
| | 8.1 mS/cm | 16 mS/cm |
| Viresolve® Pro alone | 46.5 | 26 |
| Membrane 8 device + Viresolve® Pro (Prior Art) | 487 | 1,406 |
| Membrane 7 device + Viresolve® Pro | 1,567 | > 2,390 |

**Example 10. Use of membranes according to the present invention in the protection of a downstream Virus Filter using a monoclonal antibody feed stream**

A solution of a partially purified monoclonal IgG feed (MAb III) was prepared to 6 g/L in pH 5, 50 mM acetate buffer with a conductivity of 8.5 mS/cm (using added NaCl). The MAb III had been previously shocked at a high pH (11) to generate about 4% total aggregates (as measured by SEC-HPLC). Micro filtration devices with a filtration area of 3.1 $cm^2$ were pre-molded using 3 layers of membrane and put in series at a 1:1 area ratio with Viresolve® Pro devices (EMD Millipore Corporation, Billerica, MA). Both devices were prewet and vented to remove air using only the sterile filtered buffer, pH 5, 50 mM acetate, 8.5 mS/cm. The virus membrane was also preconditioned for 10 minutes at a constant flow rate that generated a constant back pressure of 30 psi. The MAb III solution was then fed at a constant flow of 200 L/($m^2$-h) through the devices in series and the back pressure vs. time was measured. The total volume throughput was determined at an endpoint where the measured back pressure reached 30 psi. The L/$m^2$ throughput at the 30 psi cut-off was converted to kg of MAb per $m^2$ of membrane.

As can be seen from Table 8 below, the Membrane 7 provided superior protection to a virus removal filter (greater volumetric throughput) than Membrane 8.

Table 8

| | Throughput at 30 psi (kg/m2) |
|---|---|
| Viresolve® Pro alone | 0.06 |
| Membrane 8 device + Viresolve® Pro | 0.33 |
| Membrane 7 device + Viresolve® Pro | 0.66 |

**Example 11. Effect of residence time on performance of virus filter in fluid communication with Membrane 7**

In this representative experiment, the effect of residence time on performance of a virus filtration is investigated, where the virus filter is positioned downstream of Membrane 7 in a flow-through purification process. It is observed that a lower flow rate through a device containing Membrane 7 and the virus filtration step results in a higher throughput of the virus filter.

A three-layer device containing Membrane 7, having membrane area 3.1 cm2 and membrane volume 0.12 mL, is connected in a series to a virus filtration device, having a membrane area of 3.1 cm2. About 3 mg/mL of a polyclonal human IgG (Seracare) in 20 mM sodium acetate, pH 5.0 buffer, is processed through the two connected devices. The experiment is performed at two separate flow-rates, 100 and 200 LMH. A 0.22 $\mu$m sterile filter is placed between cation exchange chromatography device and the virus filtration device.

A pressure sensor is used for measuring the pressure across the assembly at the different flow rates. Normally, a pressure of about 50 psi is an indication of fouling or plugging of the virus filtration membrane. As shown in Figure 10, when the experiment is performed at a lower flow-rate (i.e., 100 LMH), more sample volume can be processed through the virus filtration membrane (i.e., higher throughput) relative to when the sample is processed at a higher flow-rate (i,e., 200 LMH). This could be attributed to longer residence time of the sample in the cation exchange chromatography device, which may result in an improvement in binding of high molecular weight IgG aggregates, thereby preventing early plugging of the virus filter.

**Example 12. Connecting several flow-through impurity removal steps into one.**

In this representative experiment, the feasibility of connecting several impurity removal steps into one simple operation, while meeting purity and yield targets, is demonstrated. This is done by connecting individual devices, namely activated carbon, an anion exchange chromatography device (e.g., ChromaSorb™), an in-line static mixer and/or a surge tank for pH change, a cation-exchange flow-through device for aggregate removal as described herein, and a virus removal device (e.g., Viresolve® Pro).

The set-up, equilibration and procedure are described below.

The flow-through purification train consists of five main unit operations: activated carbon (with optional depth filter in front), an anion exchange chromatography device (e.g., ChromaSorb), an in-line static mixer and/or surge tank for in-line pH adjustment, a cation-exchange flow-through device for aggregate removal, and a virus filtration device (e.g., Viresolve® Pro).

Figure 11 illustrates the order in which these unit operations are connected.

The necessary pumps, pressure, conductivity, and UV sensors are may additionally be included.

All devices are individually wetted at a different station, and then assembled. The devices are wetted and pre-treated according to the manufacturer's protocol. Briefly, the depth filter (A1HC grade) is flushed with 100 L/m2 of water followed by 5 volumes of equilibration buffer 1 (EB1; Protein A elution buffer adjusted to pH 7.5 with 1 M Tris-base, pH 11). 2.5 mL of activated carbon is packed into a 2.5 cm Omnifit column as described in co-pending U.S. Provisional Patent Application No. 61/575,349, filing date August 19, 2011, incorporated by reference herein, to produce MAb loading of 0.55 kg/L. The column is flushed with 10 CV water, and then equilibrated with EB1 until the pH is stabilized to pH 7.5. Two ChromaSorb devices (0.2 and 0.12 mL) are connected in series to get antibody loading of 4.3 kg/L. The devices are wetted with water at 12.5 CV/min for at least 10 min, followed by 5 DV EB1. A disposable helical static mixer (Koflo Corporation, Cary, IL) with 12 elements is used to perform in-line pH adjustments. Two 1.2 mL devices containing Membrane 7 are connected in parallel to remove aggregates, so they can be loaded to about 570 mg/mL of antibody.

They are wetted with 10 DV water, followed by 5 DV equilibration buffer 2 (EB2; EB1 Adjusted to pH 5.0 using 1 M acetic acid). The devices are further treated with 5 DV (device volumes) of EB2 + 1 M NaCl, and then equilibrated with 5 DV EB2. A 3.1 cm2 VireSolve® Pro device is wetted with water pressurized at 30 psi for at least 10 min. The flow rate is then monitored every minute until the flow rate remains constant for 3 consecutive minutes. After all the devices are wetted and equilibrated, they are connected as shown in Figure above. EB1 is run through the entire system until

all pressure readings, and pH readings are stabilized. Following equilibration, the feed (Protein A elution adjusted to pH 7.5) is passed through the flow-through train. During the run, samples are collected before the surge tank and after Viresolve® Pro to monitor IgG concentration and impurity levels (HCP, DNA, leached PrA and aggregates). After the feed is processed, the system is flushed with 3 dead volumes of EB1 to recover protein in the devices and in the plumbing.

The feed for the connected flow-through process is protein A eluate of MAb IV, produced in a batch protein A process. The natural level of aggregates in this MAb does not exceed 1%, so a special procedure was developed to increase the level of aggregates. Solution pH was raised to 11 with aqueous NaOH, with gentle stirring, and held for 1 hour. The pH was then lowered slowly to pH 5 with aqueous HCl under gentle stirring. The pH cycle was repeated 4 more times. The final level of aggregates is about 5%, mostly consisting of MAb IV dimers and trimers as measured by SEC. The feed is then dialyzed into Tris-HCl buffer, pH 7.5, conductivity about 3 mS/cm.

The MAb feed processed for this run is 102 mL of 13.5 mg/mL MAb IV at a flow rate of 0.6 mL/min.

The HCP breakthrough as a function device loading after ChromaSorb is below the upper limit of 10 ppm (Figure 12). The aggregates are reduced from 5% to 1.1% by the CEX device (Figure 13). The MAb IV yield of the connected process is 92%. The throughput on Viresolve® Pro device was >3.7 kg/m2.

Examples 13-19 demonstrate the feasibility of manufacturing compositions for removing aggregates in a flow-through mode using a cation-exchange resin or winged fibers as solid supports instead of a membrane.

**Example 13. Preparation of a polymeric strong cation-exchange (CEX) resin modified with an AMPS /DMAM grafted copolymer**

In this representative experiment, a series of cation-exchange (CEX) resins with a grafted AMPS/DMAM copolymer surface (strong CEX) were prepared with a variable density of binding groups, which are negatively charged sulfonic acid residues. The ligand density and composition of the strong cation exchange groups was controlled by the composition of reactive solution used for surface modification. In order to vary the density of the strong cation exchange groups, the charged and uncharged reactive monomers, AMPS and DMAM, were added in various molar ratios.

A 1000 mL three-necked flask with mechanical stirrer and dropping funnel is marked at a defined volume of 830 mL. In this flask, 8.25g sodium hydroxide is dissolved in 429.34g deionized water. The solution is cooled to 0°C and 13.68g AMPS is added slowly in several portions while stirring. Thereafter, 26.22g DMAM is added. The pH value of the solution is adjusted to 6.0-7.0 by the addition of 65% nitric acid and/or 1 M sodium hydroxide. 400 mL sedimented polymeric base bead resin with a mean particle size of 50 micron is added to the solution while stirring gently (120 rpm). The total volume of the reaction mixture is adjusted to 830 mL (according to mark) by the addition of deionized water. The pH value of the mixture is again adjusted to a pH of 6.0 to 7.0 by the addition of 65% nitric acid. The mixture is stirred gently (120 rpm) and heated to 40°C. A solution of 6.75g ammonium cerium(IV) nitrate and 2.96g 65% nitric acid in 15g deionized water is added quickly under vigorous stirring (220 rpm). The reaction mixture is then stirred at 120 rpm at 40°C for 3 hours.

Thereafter, the reaction mixture is poured onto a glass frit (porosity P3) and the supernatant is removed by suction. The remaining resin is washed successively with the following solutions: 3 x 400mL deionized water; 10 x 400mL 1M sulfuric acid + 0.2 M ascorbic acid; 3 x 100mL deionized water; 10 x 400mL hot deionized water (60°C); 2 x 400mL deionized water; 2 x 400mL 1 M sodium hydroxide; 2 x 100mL deionized water; during second washing step with deionized water, adjust pH to 6.5-7.0 with 25% hydrochloric acid; 2 x 400mL 70% ethanol/ 30% deionized water; 2 x 100mL deionized water; and 2 x 100mL 20% ethanol/ 80% deionized water + 150mM NaCl.

After the above washing procedure is completed, the resin is stored as a 1:1 (v/v) suspension in a solution of 20% ethanol, 80% deionized water and 150mM NaCl.

Table 9 lists the synthesized sulfonic acid containing strong CEX resins with varying molar ratios of AMPS and DMAM prepared according to this Example.

Table 9: Synthesized CEX resins prepared according to this Example

| Example | Internal Lot # | Molar quantity of DMAM [mol] | Molar quantity of AMPS [mol] | Molar ratio of DMAM/AMPS | Ionic capacity [$\mu$eq/mL] |
|---|---|---|---|---|---|
| A | 12LP-DZ105 | 0.30 | 0.075 | 4 | 32.5 |
| B | 12LP-DZ128 | 0.375 | 0.075 | 5 | 27.7 |
| C | 12LP-DZ129 | 0.45 | 0.075 | 6 | 24.7 |

(continued)

| Example | Internal Lot # | Molar quantity of DMAM [mol] | Molar quantity of AMPS [mol] | Molar ratio of DMAM/AMPS | Ionic capacity [$\mu$eq/mL] |
|---|---|---|---|---|---|
| D | 12LP-DZ119 | 0.00 | 0.075 | Pure SO3 | 12.0 |

**Example 14. Removal of aggregates from a monoclonal antibody feed using polymeric strong cation-exchange (CEX) resin modified with an AMPS /DMAM grafted copolymer**

Resin samples Lot # 12LPDZ119, 12LPDZ128, and 12LPDZ129 were packed in an Omnifit® Chromatography Column with an internal diameter of 6.6 mm to a bed height of 3 cm resulting in about 1 mL packed resin bed. An AKTA Explorer 100 (chromatography system) was equipped and equilibrated with buffers appropriate to screen these columns for flow-through chromatography (Table 10). The chromatography columns containing the resin samples 12LPDZ119, 12LPDZ128, and 12LPDZ129 were loaded onto the chromatography system with equilibration buffer. The feedstock was an IgG1 (MAbB) that was purified using ProSep® Ultra Plus Affinity Chromatography Media, and was adjusted to pH 5.0 with 2 M Tris Base. The final MAbB concentration of the protein A pool was 13.8 mg/mL,contained 2.05% aggregated product, and the conductivity was about 3.5 mS/cm. The resins were loaded at a residence time of 3 minutes and to a load density of 414 mg/mL.

Table 10: Method for performing chromatography experiments for resin samples Lot # 12LPDZ119, 12LPDZ128, and 12LPDZ129

| Method for Flow-through Chromatography Screening of Resins 12LPDZ119, 12LPDZ128, and 12LPDZ129 | | | |
|---|---|---|---|
| **Step** | **Solution** | **Residence Time (minutes)** | **Column Volumes** |
| Equilibration | 50 mM Sodium acetate pH 5 | 3 | 8 |
| Loading | 50 mM Sodium acetate pH 5 with 13.8 mg/mL mAbB | 3 | 30 (414 mg Protein/mL Resin) |
| Wash | 50 mM Sodium acetate pH 5 | 3 | 10 |
| Strip | 50 mM Sodium acetate pH, 750 mM NaCl | 3 | 5 |
| Clean in Place | 0.5 M NaOH | 3 | 5 |
| Equilibration | 50 mM Sodium acetate pH 5 | 3 | 5 |

The flow-through was collected in 2 mL fractions and assayed for total protein concentration on a NanoDrop 2000 spectrophotometer and the aggregate content was quantified by size exclusion high performance liquid chromatography (SE-HPLC). The aggregate quantification test was performed Tosoh Bioscience TSKGel G3000SWXL, 7.8 mm x 30 cm, 5 $\mu$m (Catalog # 08541) column with equilibration buffer of 0.2 M Sodium phosphate pH 7.2. The results show that the mAbB monomeric protein is collected in the flow-through fraction at high concentrations at relatively much lower cumulative protein loadings than the aggregated product.

For Lot # 12LPDZ119, 368.1 mg or approximately 88.9 % of protein was recovered at cumulative protein loading of 414 mg/mL, aggregate level was reduced from 2.05 % to 0.39 % in the flow-through fractions, and the Strip pool contained 21.2 % aggregates suggesting the resins ability to selectively retain aggregates.

For Lot # 12LPDZ128, 357.9 mg or approximately 86.4 % of protein was recovered at cumulative protein loading of 414 mg/mL, aggregate level was reduced from 2.05 % to 0.14 % in the flow-through fractions, and the Strip pool contained 13.4 % aggregates suggesting the resins ability to selectively retain aggregates.

For Lot # 12LPDZ129, 359.9 mg or approximately 86.9 % of protein was recovered at cumulative protein loading of 414 mg/mL, aggregate level was reduced from 2.05 % to 0.52 % in the flow-through fractions, and the Strip pool contained 16.8 % aggregates suggesting the resins ability to selectively retain aggregates.

Figure 14 depicts the breakthrough of mAbB monomer and aggregates for Lot # 12LPDZ119; Figure 15 depicts the breakthrough of MAbB monomer and aggregates for Lot # 12LPDZ128; and Figure 16 depicts the breakthrough of MAbB monomer and aggregates for Lot # 12LPDZ129.

As demonstrated in Figure 14, with the resin of Lot # 12LPDZ119, the MAbB concentration collected in the flow-through fractions reaches > 90 % its original load concentration with 110 mg/mL cumulative protein loading. Whereas, the aggregate level was only 0.56 % or 27.8 % of the original load concentration at 414 mg/mL cumulative protein loading. This suggests that the resin selectively retains aggregated species to high protein loadings while allowing the protein monomer (MAb) to be recovered at 88.9 % its total initial mass.

As demonstrated in Figure 15, with the resin of Lot #12LPDZ128, the mAbB concentration collected in the flow-through fractions reaches > 90 % its original load concentration with 138 mg/mL cumulative protein loading. Whereas, the aggregate level was only 0.42 % or 20.9 % of the original load concentration at 414 mg/mL cumulative protein loading. This suggests that the resin selectively retains aggregated species to high protein loadings while allowing the protein monomer to be recovered at 86.4 % its total initial mass.

As demonstrated in Figure 16, with the resin of Lot #12LPDZ129, the mAbB concentration collected in the flow-through fractions reaches > 90 % its original load concentration by 110 mg/mL cumulative protein loading. Whereas, the aggregate level was only 0.99 % or 49.2 % of the original load concentration at 414 mg/mL cumulative protein loading. This suggests that the resin selectively retains aggregated species to high protein loadings while allowing the protein monomer to be recovered at 86.9 % its total initial mass.

**Example 15. Preparation of a polymeric strong cation-exchange (CEX) resin modified with an AMPS /DMAM grafted copolymer.**

In a 250 mL glass jar, 64 ml wet cake of Toyopearl HW75-F chromatography resin was added. Next, 115g of 5M sodium hydroxide, 18.75g of sodium sulfate, and 4mL of allyl glycidyl ether (AGE) were added to the jar containing the resin. The jar was then placed in a hybridizer at 50°C overnight, with rotation at medium speed. The next day, the resin was filter drained in a sintered glass filter assembly (EMD Millipore Corporation, Billerica, MA) and the wet cake was washed with methanol and then rinsed with deionized water. In a glass vial, 10 mL wet cake of the AGE activated resin was added. To the glass vial, 0.2g of Ammonium persulfate, 0.3 g AMPS, 1.2g DMAM, and 48g of deionized water were added and the vial was heated to 60°C for 16 hours. The next day, the resin was filter drained in a sintered glass filter assembly (EMD Millipore Corporation, Billerica, MA) and the wet cake was washed with a solution of methanol and deionized water and the resin was labeled as Lot # 1712.

**Example 16. Removal of aggregates at various residence times from a monoclonal antibody feed using polymeric strong cation-exchange (CEX) resin modified with an AMPS /DMAM grafted copolymer**

The resulting resin, Lot # 1712 from Example 14 was packed in an Omnifit® Chromatography Column with an internal diameter of 6.6 mm to a bed height of 3 cm resulting in about 1 mL packed resin bed. An AKTA Explorer 100 (chromatography system) was equipped and equilibrated with buffers appropriate to screen these columns for flow-through chromatography (Similar to Example 14). The chromatography columns containing the resin sample were loaded onto the chromatography system with equilibration buffer. The feedstock was an IgG1 (mAb5) feedstock that was purified using ProSep® Ultra Plus Affinity Chromatography Media, and was adjusted to pH 5.0 with 2 M Tris Base. The final concentration of the protein A pool was diluted to 4 mg/mL,contained 5.5 % aggregated product, and a conductivity of about 3.2 mS/cm. The resin was loaded at a residence time of 1, 3, or 6 minutes and to a load density of 144 mg/mL. The strip peak fraction for the 3 minute residence timecontained 95.6 % aggregates indicating a high level of selectivity for aggregated species. The results are depicted in Table 11 below.

Table 11 depicts retention of monomer and aggregates for Lot # 1712 with MAb5 at pH 5.0 at 6, 3, or 1 minute residence time. As shown in Table 11, on average, the monomeric species can be collected at concentrations close to the feed concentration relatively early compared to the aggregated species for all residence times tested, which suggests that selectivity is relatively insensitive to flow rates.

Table 11

| Flow-through Collection | Cumulative Protein Load Density | Average of 6, 3, or 1 Minute Residence Time | 6 Minutes Residence Time | 3 Minutes Residence Time | 1 Minute Residence Time |
|---|---|---|---|---|---|
| Fraction # | (mg/mL) | % Protein in Flow-through | % Aggregates | % Aggregates | % Aggregates |
| 1 | 16 | 13.5 | 0.0% | 0.0% | 0.0% |
| 2 | 32 | 94.3 | 0.0% | 0.0% | 0.0% |

(continued)

| Flow-through Collection | Cumulative Protein Load Density | Average of 6, 3, or 1 Minute Residence Time | 6 Minutes Residence Time | 3 Minutes Residence Time | 1 Minute Residence Time |
|---|---|---|---|---|---|
| Fraction # | (mg/mL) | % Protein in Flow-through | % Aggregates | % Aggregates | % Aggregates |
| 3 | 48 | 94.4 | 0.0% | 0.0% | 0.0% |
| 4 | 64 | 95.2 | 0.0% | 0.0% | 0.0% |
| 5 | 80 | 98.3 | 0.5% | 0.0% | 0.0% |
| 6 | 96 | 100.0 | 0.7% | 0.3% | 0.0% |
| 7 | 112 | 99.3 | 1.1% | 0.9% | 2.1% |
| 8 | 128 | 100.0 | 2.3% | 1.6% | 2.8% |
| 9 | 144 | 100.0 | 3.1% | 3.6% | 4.8% |

Figure 17 depicts a chromatogram of Lot # 1712 with MAb5 at pH 5 and 3 minutes residence time. As depicted in Figure 17, the majority of the product is collected in the flow-through and this is indicated by the relatively quick breakthrough of protein UV trace. The strip peak size generally varies based on the conditions and total mass loaded but it is relatively enriched with aggregate species at 95.6 %, compared to the load material which had only 5.5 % aggregates.

**Example 17. Purification of a monoclonal antibody using Protein A affinity chromatography followed by the use of a chromatography resin**

In a representative experiment described herein, a monoclonal antibody was purified using Protein A affinity chromatography followed by the use of a chromatography resin according to the present invention. The results of this experiment demonstrate an unexpected finding that the methods did not require an increase in conductivity or the use of dilutions, when run in a flow-through mode.

An IgG1 (MAb5) was expressed in a cell culture of Chinese Hamster Ovary (CHO) cells. The cell culture was clarified by two stage depth filtration followed by sterile filtration. The clarified cell culture containing 0.5 mg/mL mAb5 was first purified using ProSep® Ultra Plus Affinity Chromatography Media (Protein A). The Protein A chromatography elution buffer used was 100 mM acetic acid. The Protein A elution pool was adjusted to a pH of 5.0 using 2 M Tris base and the resulting solution had a conductivity of about 3.5 mS/cm. The Resin Lot # 1712 was run in flow-through mode according to method described herein at 3 minute residence time and flow-through fractions were collected and small aliquots were reserved for assaying.

The flow-through fractions were pooled, adjusted to pH 7.5, and run in flow-through mode using either ChromaSorb, which is a salt-tolerant anion-exchange membrane adsorber, which was loaded to 5 kg/L or Fractogel TMAE, which is an anion exchange resin, which was loaded to 150 mg/mL. The fractions were assayed for protein concentration, Aggregate level, leached protein A, and Chinese Hamster Ovary Proteins (CHOP). The results are depicted in Table 12 below.

Table 12

| | Step / Protein Load | % Recovery for step | % Aggregate s in in pool | Leached Protein A in pool (ppm) | CHOP in pool (ppm) |
|---|---|---|---|---|---|
| Step 1 | Protein A affinity chromatography pool | 97 | 5.40 | 251 | 24000 |
| | Resin Lot # 1712 run in flow-through mode as described herein | | | | |

(continued)

| Step 2 | Cumulative Protein Load Density (mg/mL) | % Protein in Flow-through | % Aggregates in Flow-through | Leached Protein A in Flow-through (ppm) | CHOP in Flow-through (ppm) |
|---|---|---|---|---|---|
| | 16 | 14 | 0.00 | 0.4 | 1 |
| | 32 | 94 | 0.00 | 1.8 | 1300 |
| | 48 | 94 | 0.00 | 1.9 | 4400 |
| | 64 | 95 | 0.00 | 2.1 | 9300 |
| | 80 | 98 | 0.50 | 2.4 | 19400 |
| | 96 | 100 | 0.70 | 2.5 | 24800 |
| | 112 | 99 | 1.10 | 3.1 | 25500 |
| | CIEx FT Pool | >90 % | <0.5% | 0.8 (ppm) | 11500 (ppm) |
| | | | | | |
| Step 3 or Step 3 | ChromaSorb anion exchange membrane adsorber | >90 % | <0.5% | NA | 60 |
| | Fractogel TMAE | >90 % | <0.5% | NA | 200 |

Typically, a chromatographic purification process involves a traditional bind- and-elute cation exchange chromatography as the step prior to anion exchange chromatography and further requires a dilution step or a buffer exchange step in order to reduce the conductivity to a level that is suitable for anion exchange flow-through chromatography. However, as shown in Table 12, the processes described herein using a cation exchange media according to the present invention do not require an increase in conductivity in order to operate, and consequently, do not require a dilution step or a buffer exchange step prior to the purification step.

**Example 18. Preparation of strong cation-exchange (CEX) winged fiber modified with an AMPS /DMAM grafted copolymer**

In this representative experiment, cation-exchange winged fibers were used as the solid support.

In a 1L glass jar, 20g of dry Nylon multi-lobed, or winged, fibers were combined with 400 g of 4M sodium hydroxide, 24 g of sodium sulfate, and 160 mL of allyl glycidyl ether (AGE). The jar was then placed in a hybridizer at 50°C overnight rotating at medium speed. The following day, the fibers were filtered in a sintered glass filter assembly and the fibers were then washed with methanol and rinsed with Milli-Q water. A day later, the fibers were washed with water, followed by methanol, and then water again, suctioned to a dry cake and dried in vacuum oven at 50°C for 1 day. The resulting sample was labeled Sample #1635. In three separate glass vials, 2 grams dry cake of Sample # 1635, AGE activated fibers, were weighed out and added to a glass vial for additional modification by grafting. To the glass vial, ammonium persulfate, AMPS, DMAM, and deionized water were added in amounts specified in Table 13 and the vial was heated to 60°C for 16 hours with continuous rotation. The following day, the fiber samples were filtered in a sintered glass filter assembly and the wet cake was washed with a solution of deionized water. The vials containing the fibers were labeled as Lot # 1635-1, 1635-2, and 1635-5. Next, Lot #1635-5 was titrated for small ion capacity, which was found to be about 28 $\mu$mol/mL. It was then assumed that samples #1635-1 and #1635-2 also had small ion capacity less than 28$\mu$mol/mL.

Table 13

| Ingredients | #1635-1 | #1635-2 | #1635-5 |
|---|---|---|---|
| Fibers (g) | 2.0 | 2.0 | 2.0 |
| Ammonium persulfate (g) | 0.18 | 0.18 | 0.18 |
| AMPS (g) | 0.48 | 0.60 | 0.72 |

(continued)

| Ingredients | #1635-1 | #1635-2 | #1635-5 |
|---|---|---|---|
| DMAM (g) | 0.48 | 0.60 | 0.72 |
| Water (g) | 28.86 | 28.62 | 28.38 |

**Example 19: Removal of aggregates from a monoclonal antibody feed using strong cation-exchange (CEX) winged fibers modified with an AMPS /DMAM grafted copolymer.**

The resulting modified winged fibers, Lot # 1635-1, #1635-2, #1635-5 from Example 17 were packed in an Omnifit® Chromatography Column with an internal diameter of 6.6 mm to a bed height of 3 cm resulting in about 1 mL packed fiber bed. An AKTA Explorer 100 (chromatography system) was equipped and equilibrated with buffers appropriate to screen these columns for flow-through chromatography (Similar to Example 13). The chromatography columns containing the winged fiber samples were loaded onto the chromatography system with equilibration buffer. The feedstock was an IgG1 (mAb5) feedstock that was purified using protein A affinity chromatography, and was adjusted to pH 5.0 with 2 M Tris Base. The final concentration of the protein A pool was 4 mg/mL and contained 5.5 % aggregated or HMW product. The columns packed with fiber Lot # 1635-1 and Lot # 1635-2 were loaded to a mass loading of 64 mg/mL and the column packed with fiber Lot 1635-5 was loaded to a mass loading of 80 mg/mL. The results are depicted in Table 14 below.

Table 14

| Cumu-lative protein load mg/mL | Fiber Lot #1635-1 | | | Fiber Lot #1635-2 | | | Fiber Lot #1635-5 | | |
|---|---|---|---|---|---|---|---|---|---|
| | Monomer (%) | Dimers (%) | LMW (%) | Monomer (%) | Dimers (%) | LMW (%) | Monomer (%) | Dimers (%) | LMW (%) |
| 8 | 0.0 | 0.0 | 0.0 | 0.0 | 0.0 | 0.0 | 0.0 | 0.0 | 0.0 |
| 16 | 85.8 | 0.0 | 14.0 | 86.0 | 0.0 | 15.6 | 1.7 | 0.0 | 10.2 |
| 24 | 84.3 | 0.0 | 15.6 | 85.6 | 0.0 | 14.8 | 51.8 | 0.0 | 13.4 |
| 32 | 83.2 | 0.6 | 16.0 | 83.0 | 0.0 | 17.1 | 85.6 | 0.0 | 15.1 |
| 40 | 79.4 | 2.4 | 17.7 | 81.1 | 0.0 | 14.9 | 86.5 | 0.0 | 13.9 |
| 48 | 79.6 | 4.4 | 15.8 | 82.5 | 1.5 | 15.9 | 83.3 | 0.0 | 15.8 |
| 56 | 82.4 | 4.4 | 12.8 | 79.4 | 2.1 | 18.3 | 87.1 | 0.0 | 13.1 |
| 64 | 81.1 | 4.5 | 14.2 | 83.1 | 3.1 | 14.7 | 85.4 | 2.3 | 13.6 |
| 72 | N/A* | N/A | N/A | N/A | N/A | N/A | 85.7 | 4.0 | 13.6 |
| 80 | N/A | N/A | N/A | N/A | N/A | N/A | 85.1 | 5.1 | 13.8 |
| Strip Peak | 38.0 | 63.5 | 0.0 | 54.4 | 35.0 | 11.0 | 51.3 | 43.0 | 5.6 |

*N/A = Not applicable

The specification is most thoroughly understood in light of the teachings of the references cited within the specification which are hereby incorporated by reference. The embodiments within the specification provide an illustration of embodiments in this invention and should not be construed to limit its scope. The skilled artisan readily recognizes that many other embodiments are encompassed by this invention. All publications and inventions are incorporated by reference in their entirety. To the extent that the material incorporated by reference contradicts or is inconsistent with the present specification, the present specification will supercede any such material. The citation of any references herein is not an admission that such references are prior art to the present invention.

Unless otherwise indicated, all numbers expressing quantities of ingredients, cell culture, treatment conditions, and so forth used in the specification, including claims, are to be understood as being modified in all instances by the term "about." Accordingly, unless otherwise indicated to the contrary, the numerical parameters are approximations and

may vary depending upon the desired properties sought to be obtained by the present invention. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the following claims.

Many modifications and variations of this invention can be made without departing from its spirit and scope, as will be apparent to those skilled in the art. The specific embodiments described herein are offered by way of example only and are not meant to be limiting in any way. It is intended that the specification and examples be considered as exemplary only, with a true scope and spirit of the invention being indicated by the following claims.

Embodiments of the invention may include features of the following enumerated paragraphs ("paras"):

1. A flow-through chromatography method of separating a monomeric protein of interest from protein aggregates in a sample, the method comprising contacting the sample with a solid support comprising one or more cation exchange binding groups attached thereto, at a density of about 1 to about 30 mM, wherein the solid support selectively binds protein aggregates, thereby to separate the monomeric protein of interest from protein aggregates.

2. The method according to para 1, wherein the solid support is:

a) selected from a chromatographic resin, a membrane, a porous bead, a porous monolith, a winged fiber, a woven fabric and a non-woven fabric; or
b) a porous polyvinylether polymeric bead or a porous crosslinked polymethacrylate polymer bead; or
c) is selected from a chromatographic resin or a porous bead; or
d) is a chromatographic resin or porous bead that comprises a mean particle size of between about 10 and about 500 microns; or
e) is a chromatographic resin or porous bead that comprises a mean particle size of between about 20 and about 140 microns; or
f) is a chromatographic resin or porous bead that comprises a mean particle size of between about 30 and about 75 microns; or
g) is a chromatographic resin or porous bead that comprises a mean particle size of about 50 microns.

3. The method according to para 1 or para 2, wherein the protein aggregates are:

a) lower order protein aggregates; or
b) lower order protein aggregates selected from the group consisting of dimers, trimers, and tetramers; or
c) high molecular weight protein aggregates; or
d) higher molecular weight aggregates that are pentamers and higher.

4. The method according to any one of paras 1 to 3, wherein the one or more cation exchange group is selected from the group consisting of a sulfonic group, a sulfate group, a phosphonic group, a phosphoric group, and a carboxylic group.

5. The method according to any one of paras 1 to 4, wherein the monomeric protein of interest is:

a) an antibody; or
b) a monoclonal antibody; or
c) a recombinant protein.

6. The method of para 1, wherein the sample, prior to aggregate removal, is purified by one or more of flow-through adsorbers selected from the group containing anion-exchange media and activated carbon; and optionally
wherein the aggregate removal is connected directly to prior purification steps without an intermediate holding container; and/or
wherein concentration of the protein of interest in the effluent is at least 80% of the concentration of the protein of interest in a).

7. The method according to any one of the preceding paras, wherein the solid support binds protein aggregates relative to monomers at a selectivity greater than about 10.

8. A flow-through chromatography method of reducing the concentration of protein aggregates in a sample, optionally

according to any one of paras 1 to 7, the method comprising:

a) providing a sample comprising a protein of interest and protein aggregates;
b) contacting the sample with a solid support comprising one or more cation exchange binding groups attached thereto, at a density of about 1 to about 30 mM; and
c) collecting a flow-through effluent of the sample,

wherein the concentration of protein aggregates in the effluent is reduced by at least 50% relative to the concentration of the aggregates in a), thereby to reduce the concentration of the protein aggregates in the sample.

9. A flow-through process for purifying a target molecule from a Protein A eluate comprising the steps of:

a) contacting the eluate recovered from a Protein A chromatography column with activated carbon;
b) contacting a flow-through sample from step a) with an anion exchange chromatography media; and
c) contacting a flow-through sample from step b) with a solid support comprising one or more cation exchange binding groups attached thereto, at a density of about 1 to about 30 mM; and
d) obtaining a flow-through sample from step c) comprising the target molecule,

wherein the eluate flows continuously through steps a) - c) and wherein level of one or more impurities in the flow-through sample after step c) is lower than the level in the eluate in step a).

10. The flow-through process of para 9, wherein:

a) the process further comprising subjecting the flow-through sample from step (c) to virus filtration; and/or
b) the process further comprising use of an in-line static mixer and/or a surge tank between steps b) and c) to change pH; and/or
c) the process employs a single skid; and/or
d) the eluate from the Protein A chromatography column is subjected to virus inactivation prior to contacting with activated carbon; and/or
e) steps a) - c) may be performed in any order.

11. A flow-through purification process for purifying a target molecule from a Protein A eluate, the process comprising contacting the eluate with a cation exchange media and at least one other media selected from the group consisting of activated carbon, anion exchange media and virus filtration media, wherein the flow of the eluate is continuous, and wherein the cation exchange media comprises one or more cation exchange binding groups at a density of about 1 to about 30 mM.

12. A flow-through process for increasing the purity of a target molecule in a Protein A eluate comprising the steps of:

a) contacting the eluate recovered from a Protein A chromatography column with a solid support comprising one or more cation exchange binding groups attached thereto, at a density of about 1 to about 30 mM; and
b) obtaining a flow-through sample from step a) comprising the target molecule,

wherein the level of aggregates in the flow-through sample is lower than the level of aggregates in the Protein A eluate, thereby increasing the purity of the target molecule.

13. A flow-through process for purifying a target molecule from a Protein A eluate, wherein the process is performed at ionic conductivity less than or equal to about 10 mS/cm.

14. The process according to either para 12 or 13, wherein the target molecule is a monoclonal antibody.

15. The process according to any one of paras 9 to 14, including the method of any one of paras 1 to 8.

16. A polymer:

a) comprising the following chemical structure:

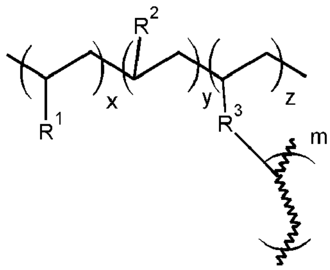

wherein $R^1$ is a cation-exchange group; $R^2$ is any aliphatic or aromatic organic residue that does not contain a charged group; $R^3$ is any uncharged aliphatic or aromatic organic linker between any two or more polymeric chains; x, y, and z are average molar fractions of each monomer in the polymer, wherein y>x; and wherein I m denotes a similar polymer chain attached at the other end of the linker; or

b) comprising the following chemical structure:

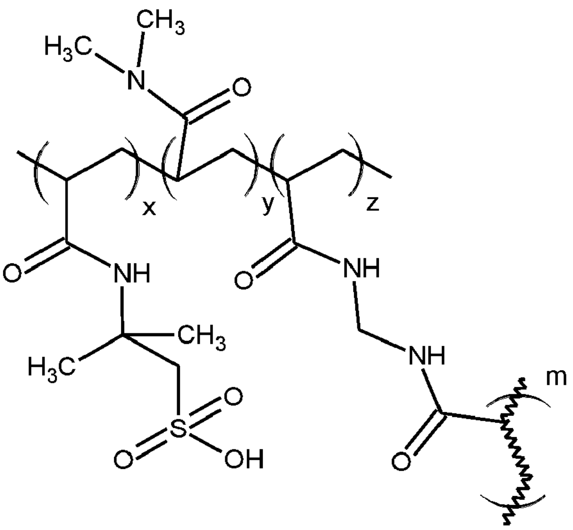

wherein x, y, and z are average molar fractions of each monomer in the polymer, wherein y>x; and wherein m represents a second polymer; or

c) comprising the following chemical structure:

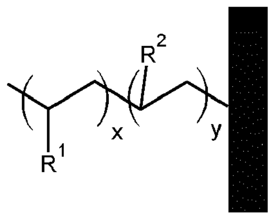

wherein $R^1$ is a cation-exchange group; $R^2$ is any aliphatic or aromatic organic residue that does not contain a charged group; and x and y are average molar fractions of each monomer in the polymer, where y>x, and wherein the polymer is grafted via covalent linkage onto a solid support, shown as the rectangle; or

d) comprising the following chemical structure, wherein the polymer includes two or more monomers and the polymer is grafted via a linkage onto a chromatography resin:

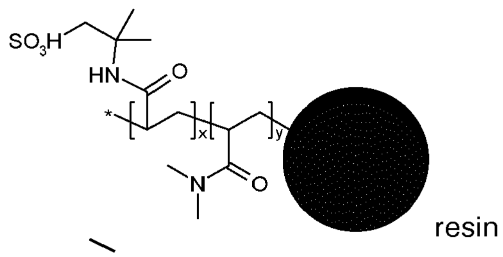

wherein x and y are average molar fractions of each monomer in the polymer, wherein y > x.

17. The polymer according to para 16 part a) or b), wherein the polymer is attached to a solid support.

18. The polymer according to para 16 or para 17 used in a method according to any one of paras 1 to 8 or a process according to any one of paras 9 to 15.

## Claims

**1.** A polymer comprising the following chemical structure:

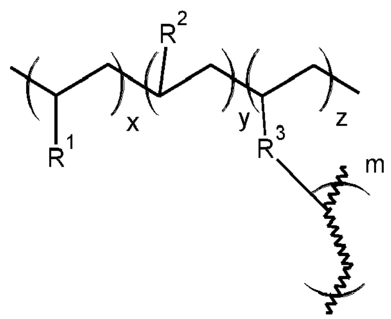

wherein $R^1$ is a cation-exchange group; $R^2$ is any aliphatic or aromatic organic residue that does not contain a charged group; $R^3$ is any uncharged aliphatic or aromatic organic linker between any two or more polymeric chains; x, y, and z are average molar fractions of each monomer in the polymer, wherein y>x; and wherein m denotes a similar polymer chain attached at the other end of the linker.

**2.** A polymer comprising the following chemical structure:

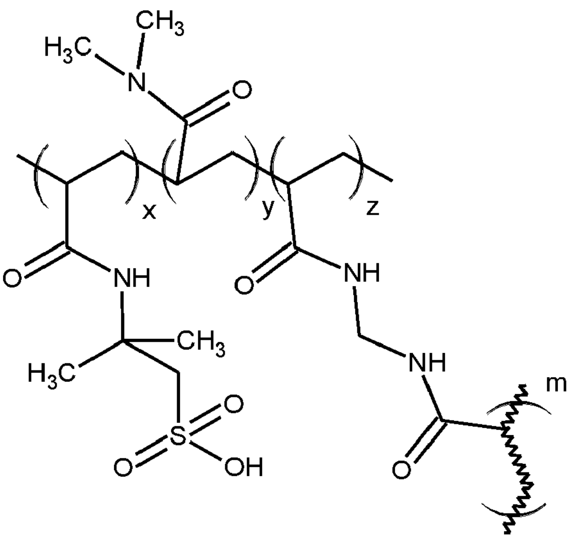

wherein x, y, and z are average molar fractions of each monomer in the polymer, wherein y>x; and wherein *m* represents a second polymer.

**3.** The polymer of claim 1, wherein said polymer is attached to a solid support.

**4.** The polymer attached to a solid support of claim 3, wherein said attachment is by one or more of adsorption, grafting or coating.

**5.** The polymer attached to a solid support of claim 4, wherein said attachment is via an *in situ* polymerization reaction that incorporates the binding group into a cross-linked coating applied onto the solid support.

**6.** The polymer of claim 2, wherein said polymer is attached to a solid support.

**7.** The polymer attached to a solid support of claim 6, wherein said attachment is by one or more of adsorption, grafting or coating.

**8.** The polymer attached to a solid support of claim 7, wherein said attachment is via an *in situ* polymerization reaction that incorporates the binding group into a cross-linked coating applied onto the solid support.

**9.** A polymer comprising the following chemical structure:

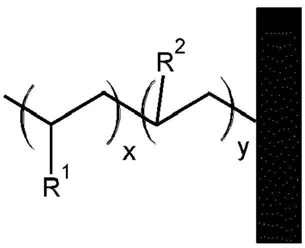

wherein $R^1$ is a cation-exchange group; $R^2$ is any aliphatic or aromatic organic residue that does not contain a charged group; and x and y are average molar fractions of each monomer in the polymer, where y>x, and wherein the polymer is grafted via covalent linkage onto a solid support, shown as the rectangle.

**10.** A polymer comprising the following chemical structure, wherein the polymer includes two or more monomers and the polymer is grafted via a linkage onto a chromatography resin:

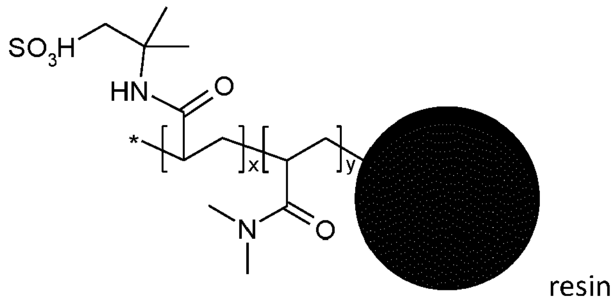

wherein x and y are average molar fractions of each monomer in the polymer, wherein y > x.

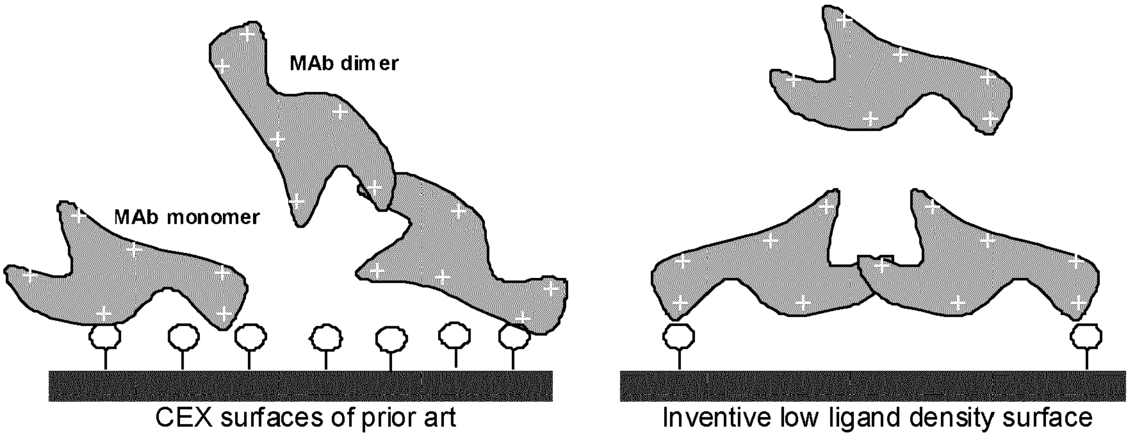


Figure 1

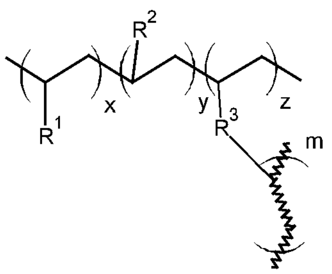

Figure 2A

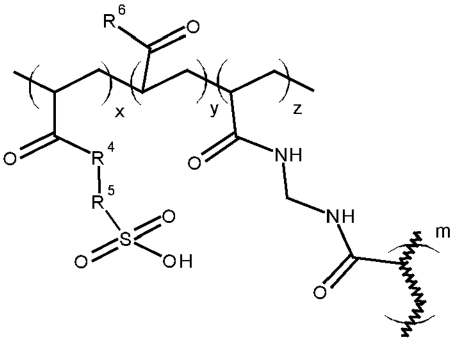

Figure 2B

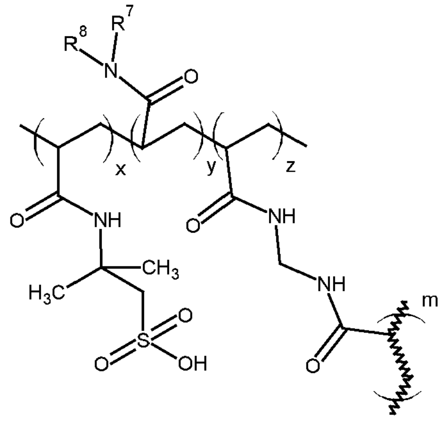

Figure 2C

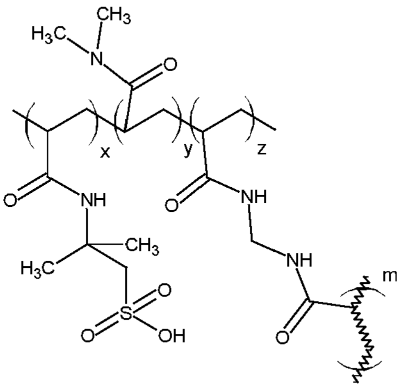

Figure 2D

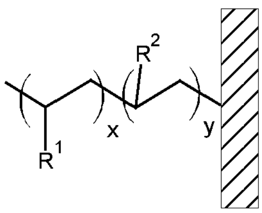

Figure 2E

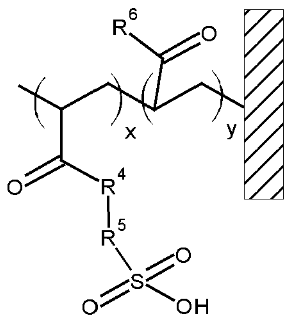

Figure 2F

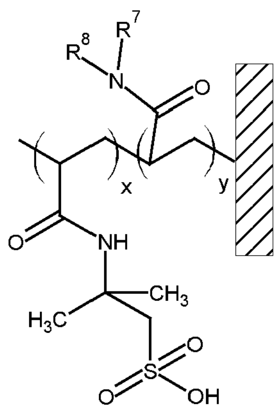

Figure 2G

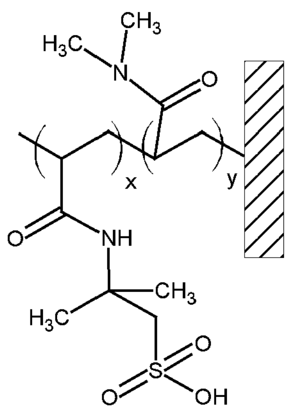

Figure 2H

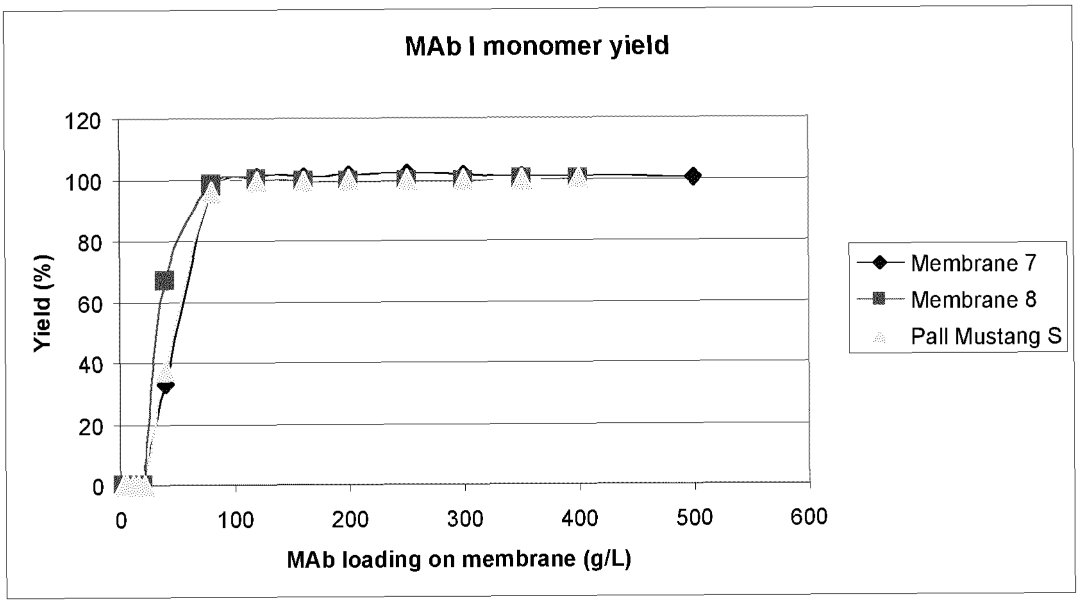


Figure 3A

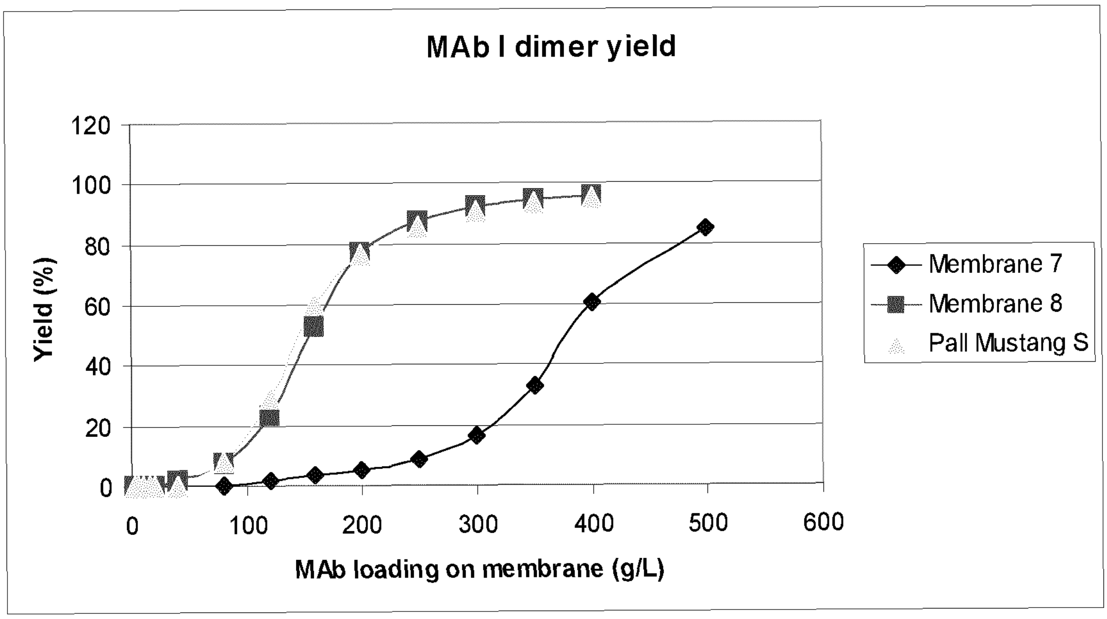


Figure 3B

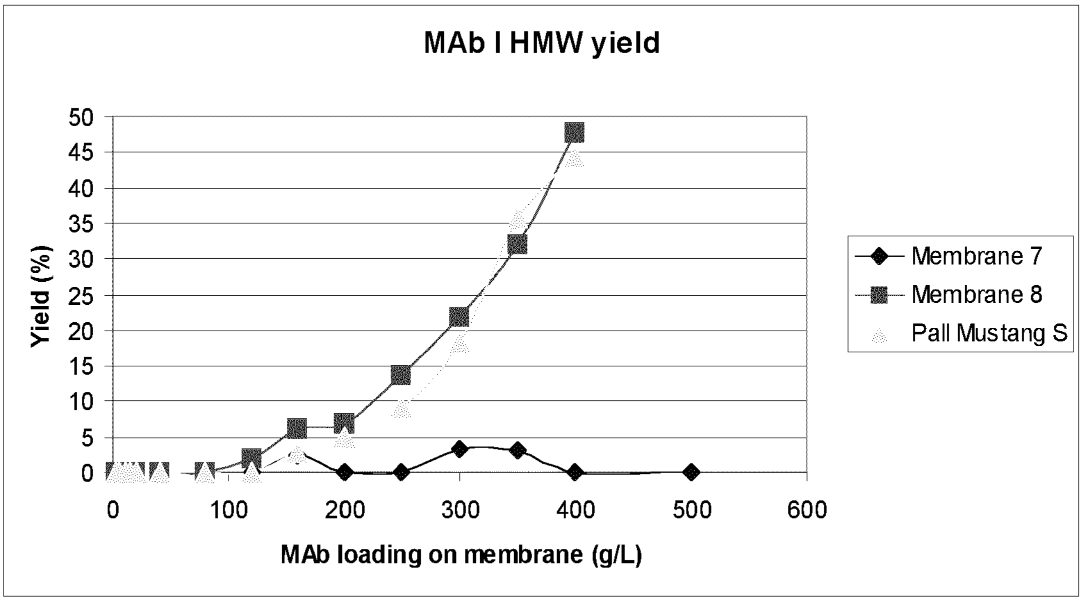


Figure 3C

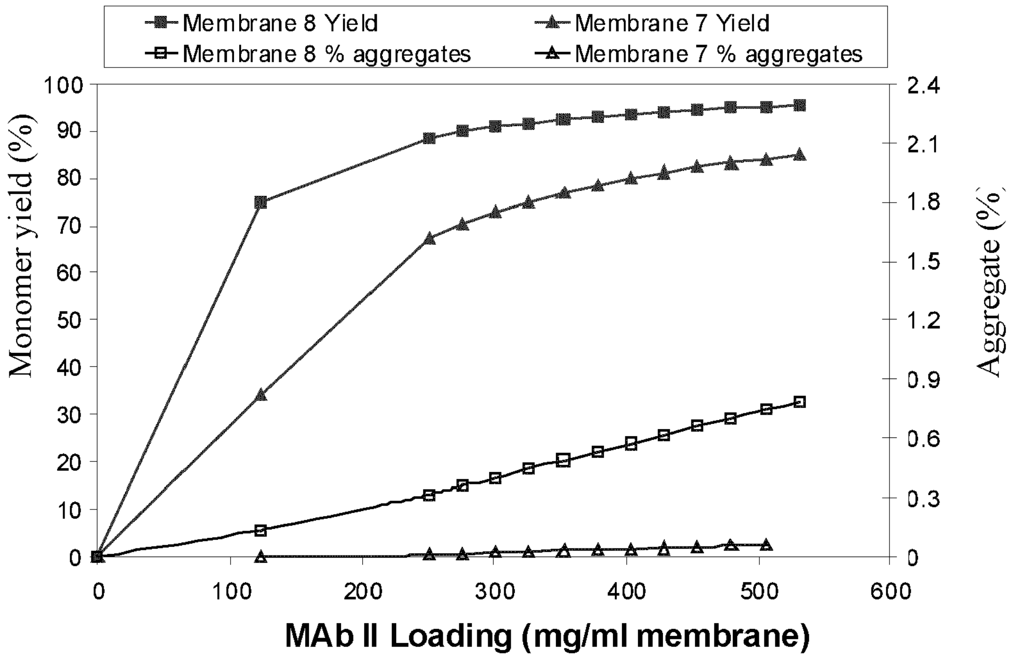


Figure 4A

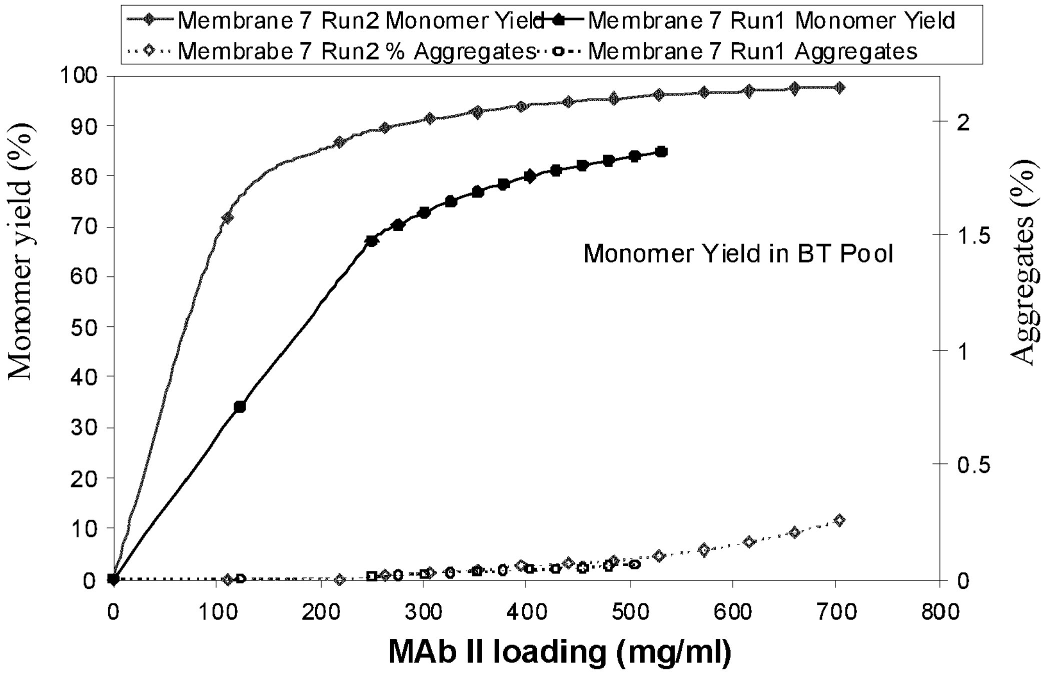


Figure 4B

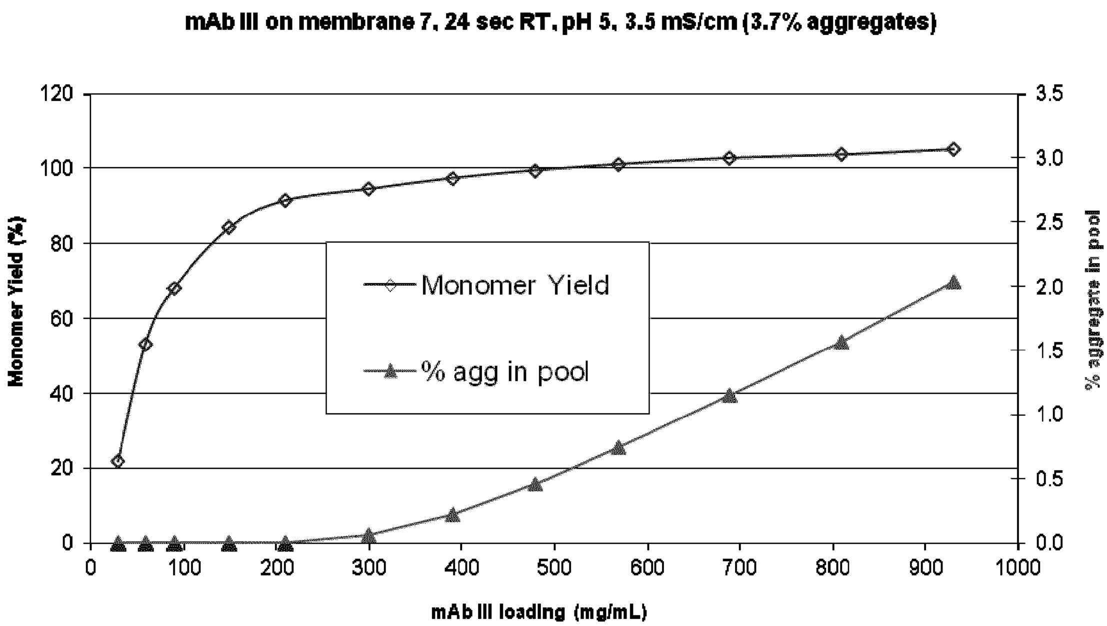


Figure 5

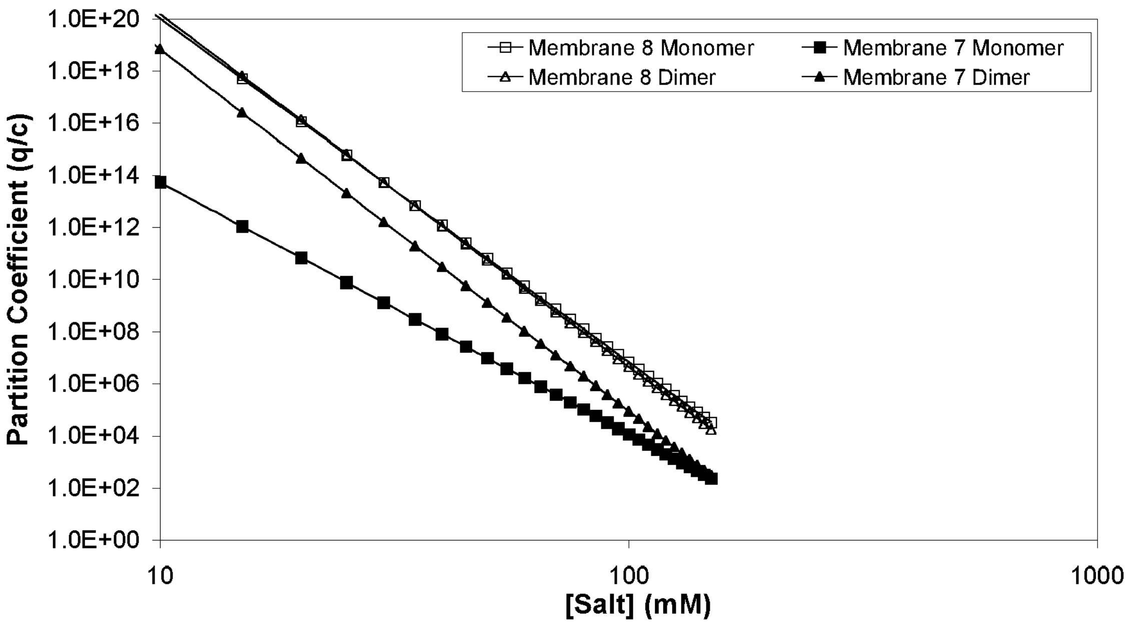


Figure 6A

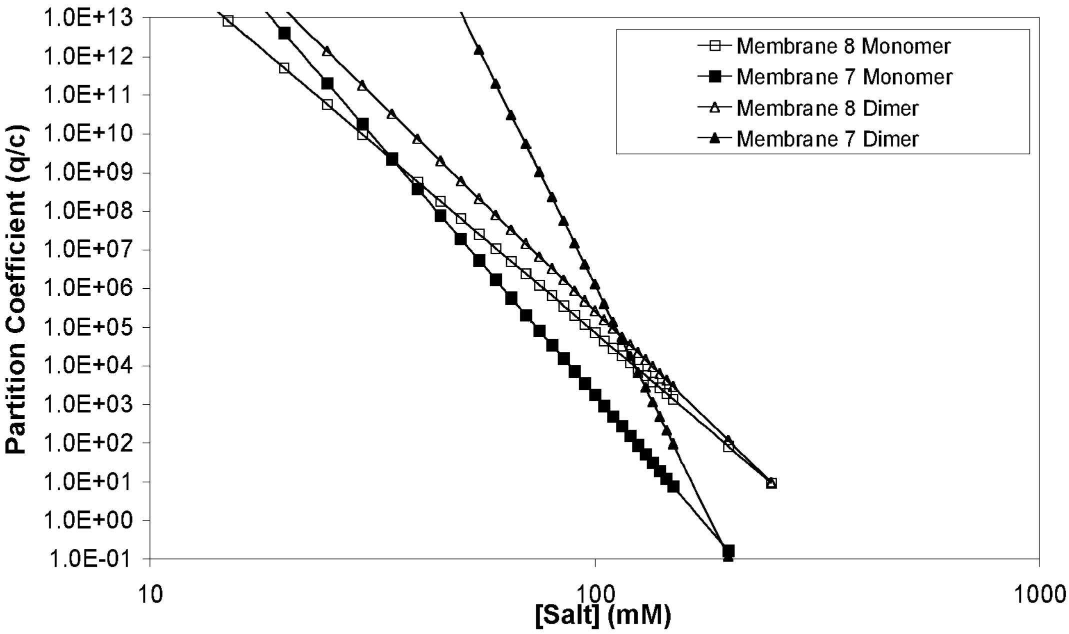


Figure 6B

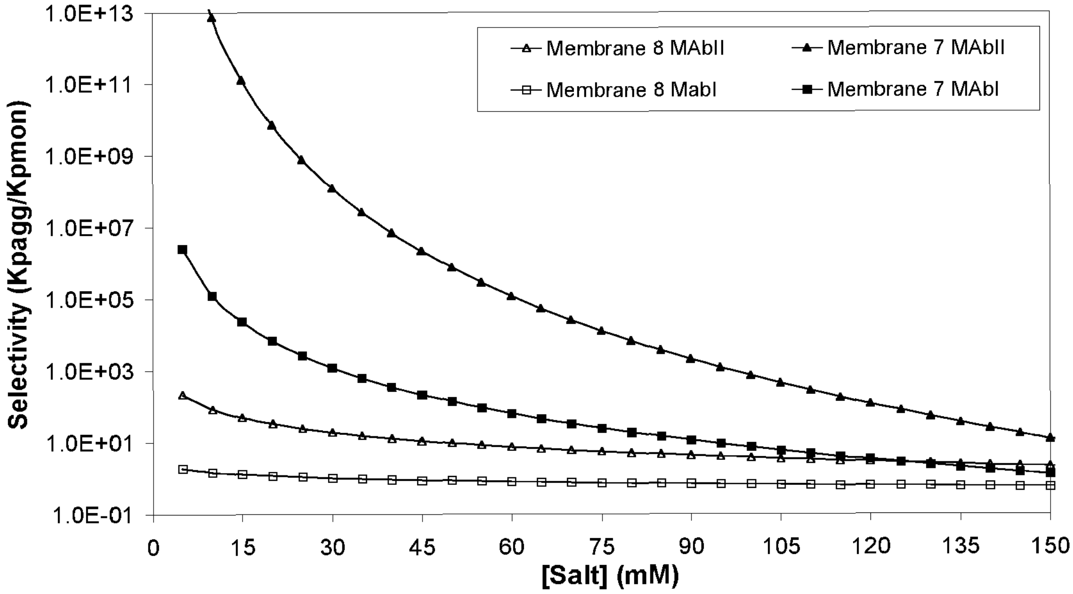


Figure 7

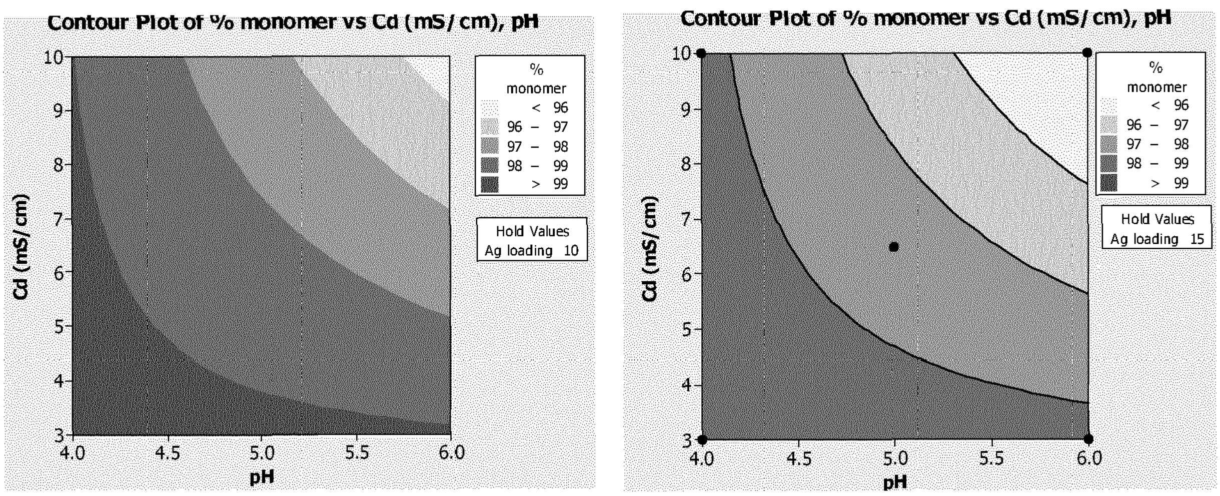


Figure 8

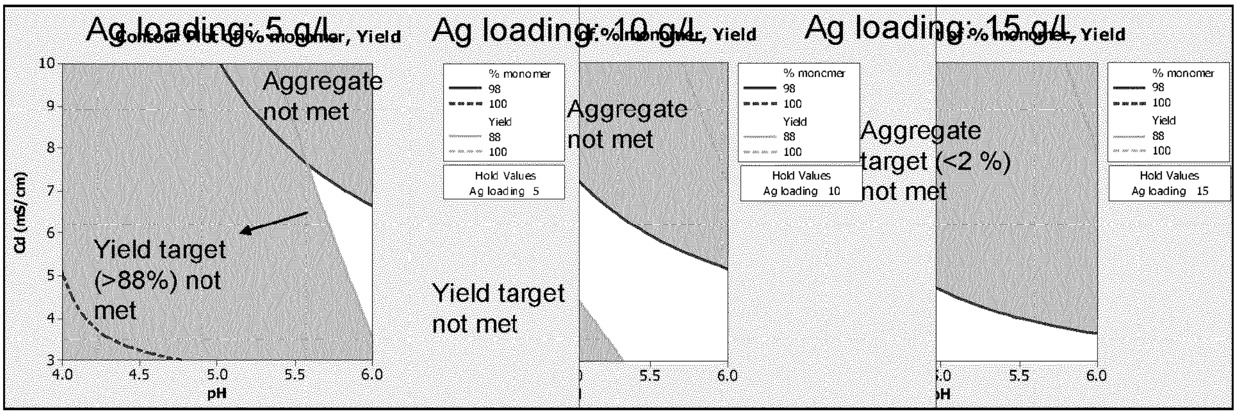


Figure 9

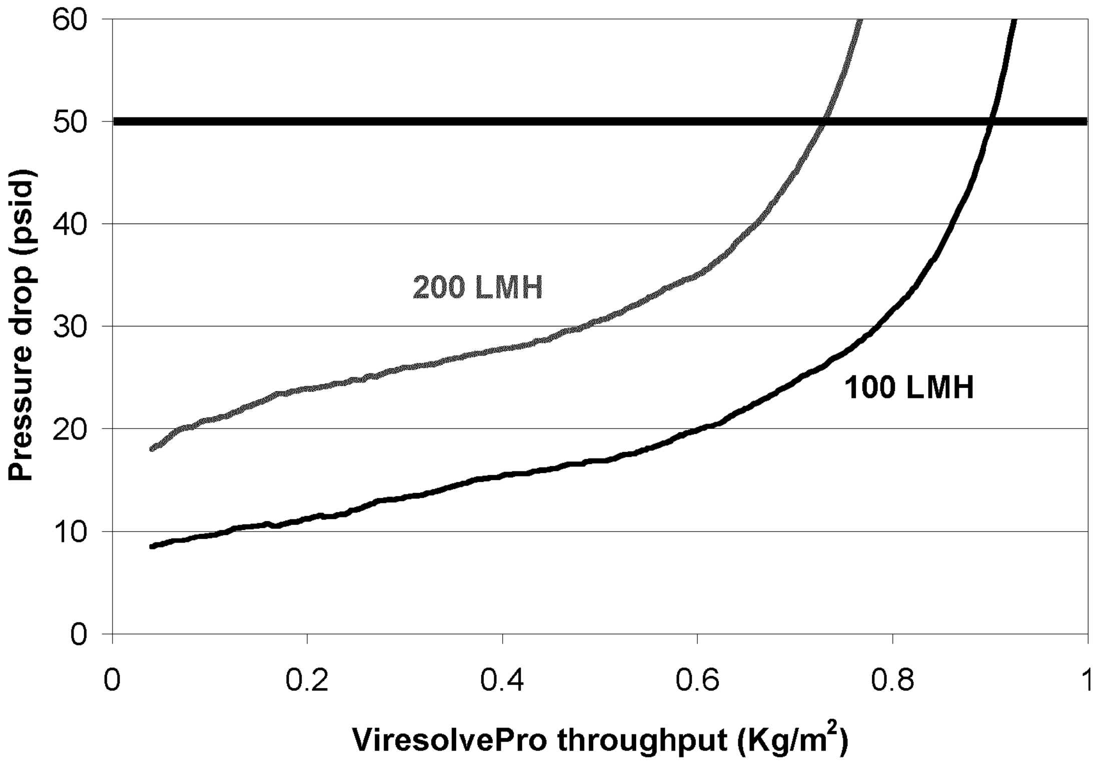


Fig. 10.

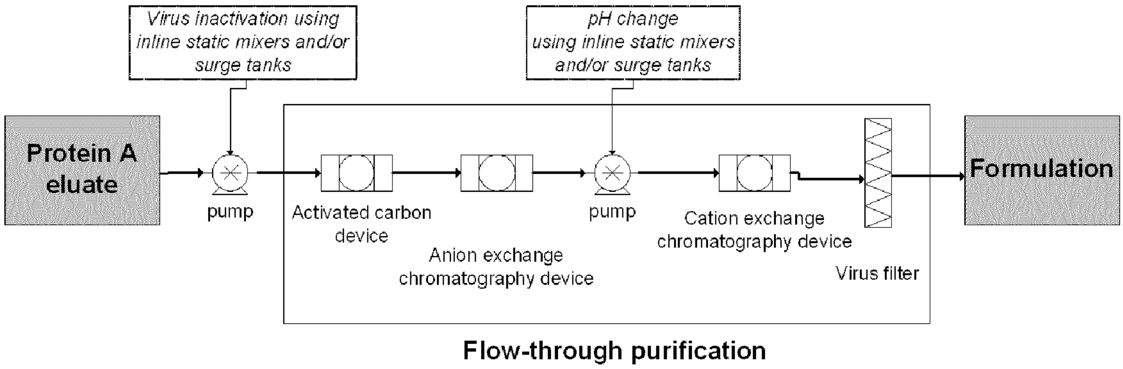
Virus inactivation using inline static mixers and/or surge tanks
pH change using inline static mixers and/or surge tanks
Protein A eluate
pump
Activated carbon device
Anion exchange chromatography device
pump
Cation exchange chromatography device
Virus filter
Formulation
Flow-through purification

Fig. 11.

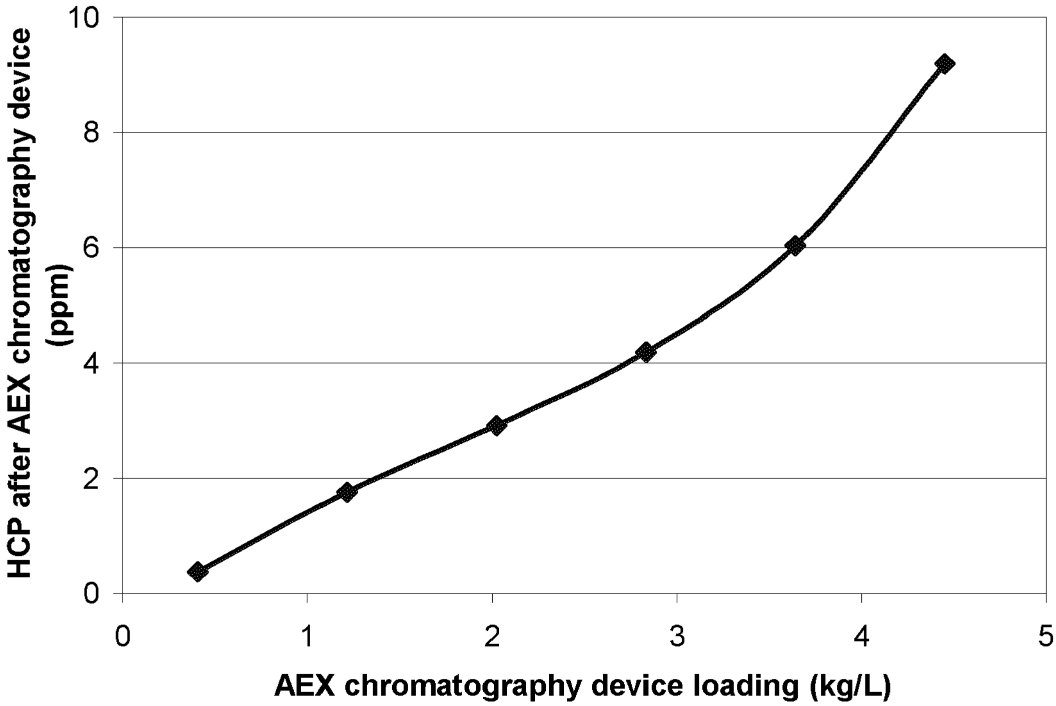
HCP after AEX chromatography device (ppm)
0
2
4
6
8
10
0
1
2
3
4
5
AEX chromatography device loading (kg/L)

Fig. 12

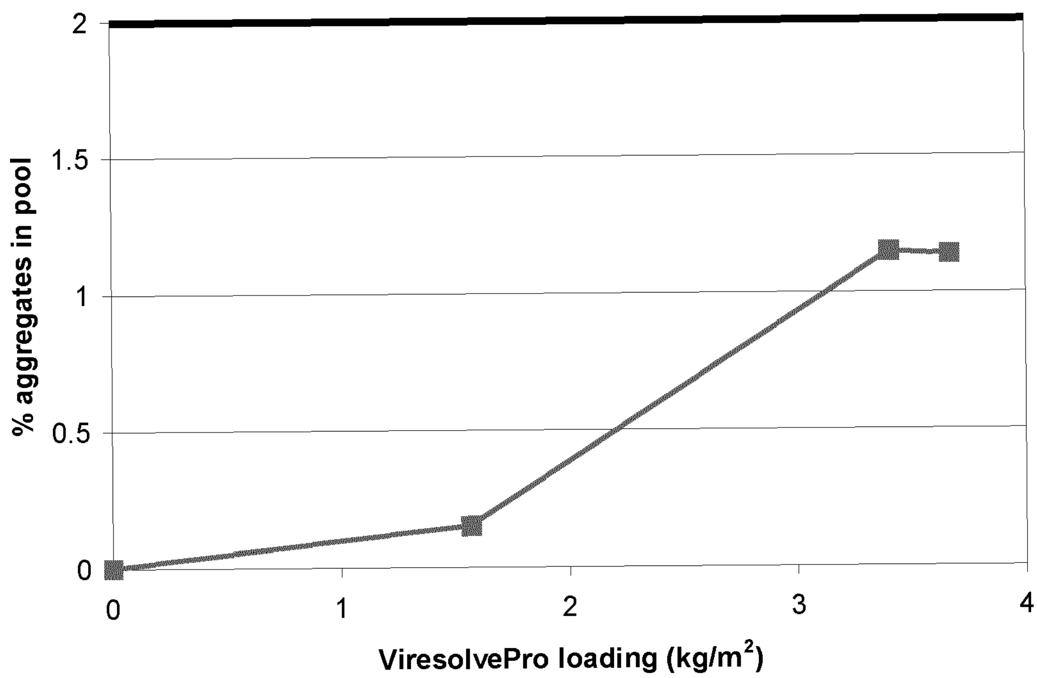


Figure 13.

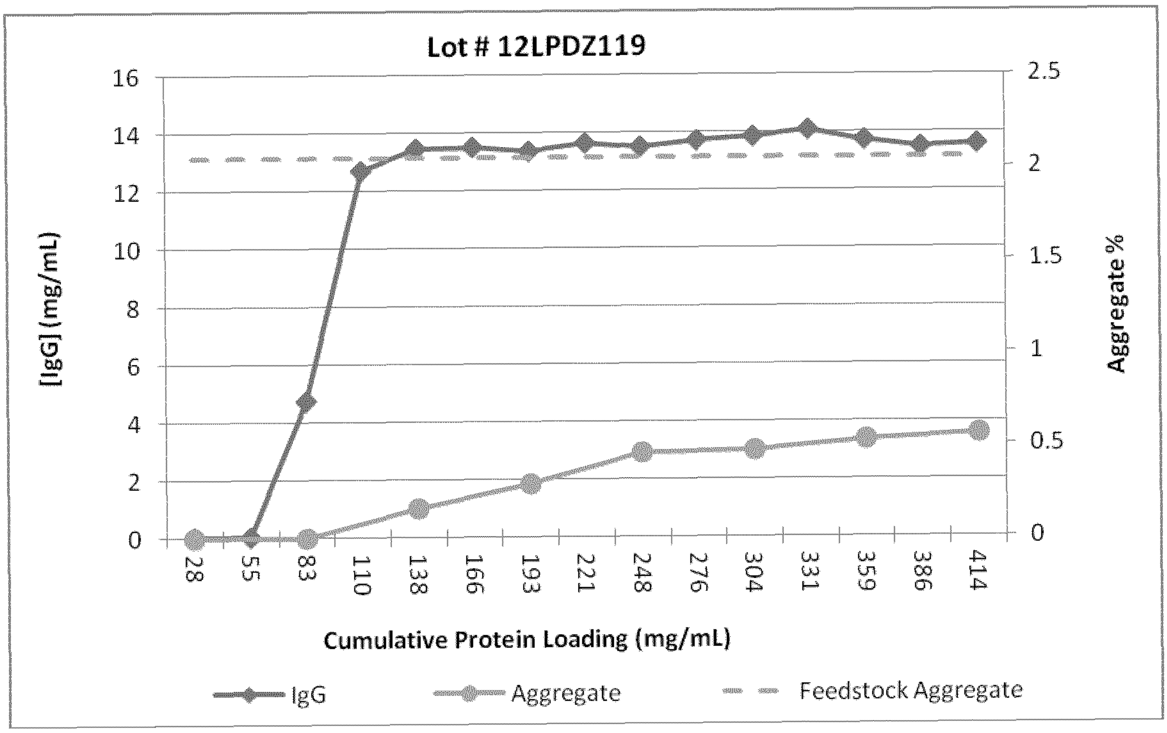


Figure 14

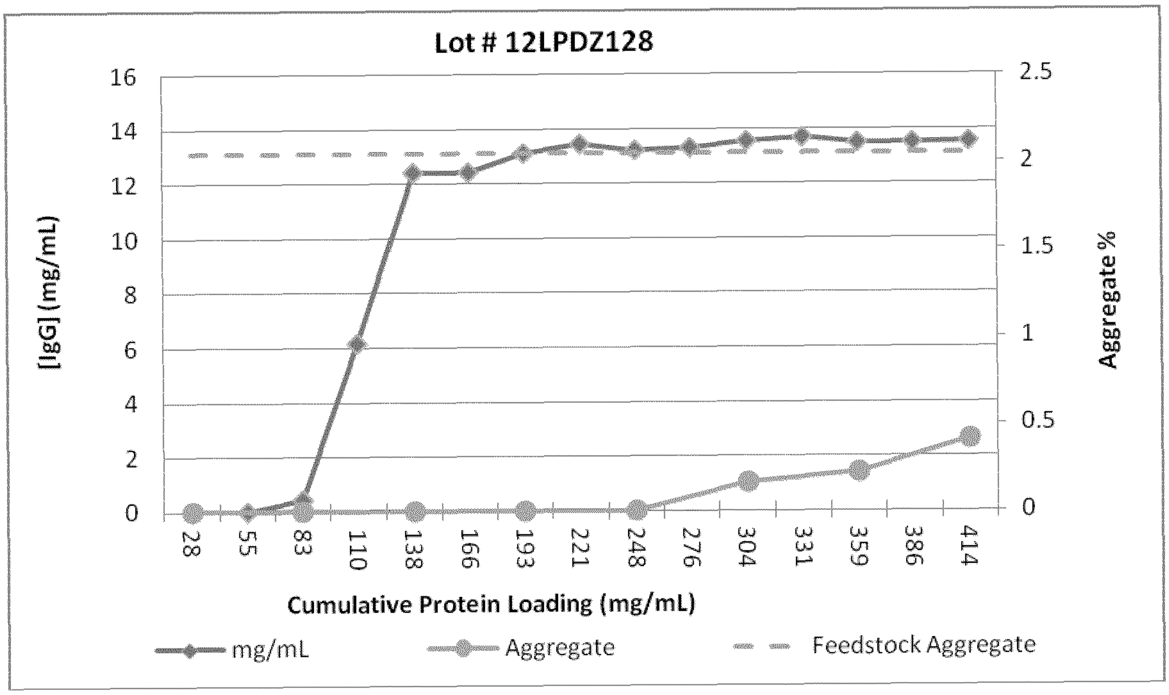


Figure 15

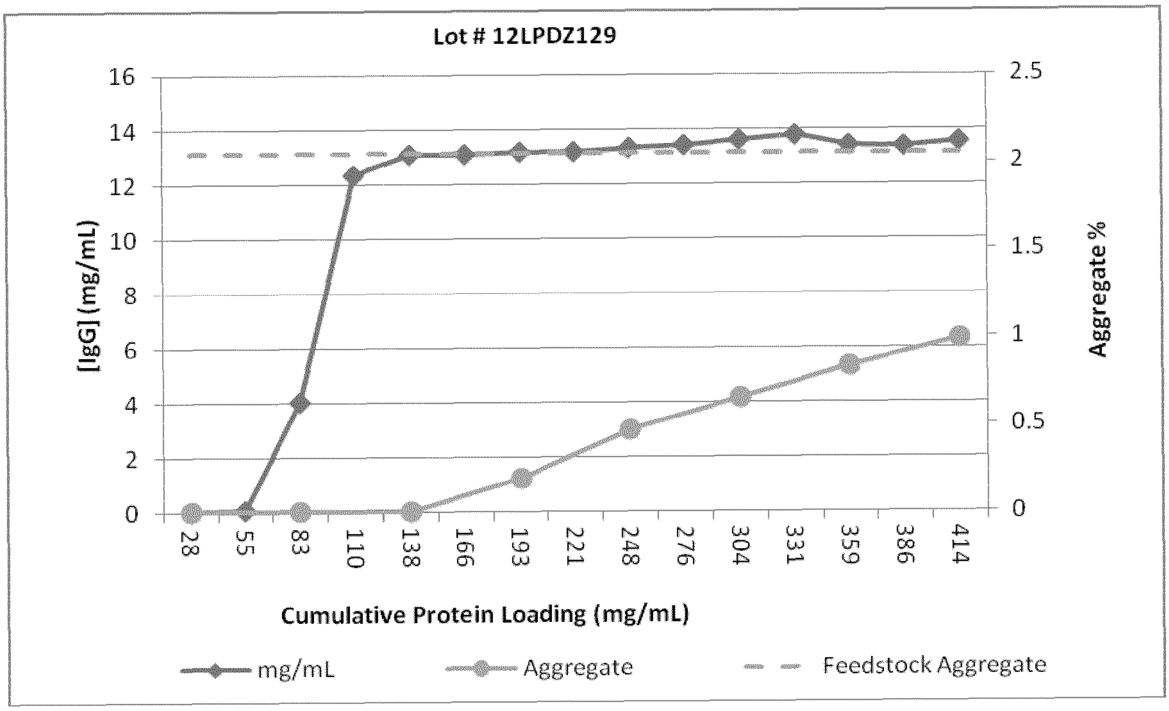


Figure 16

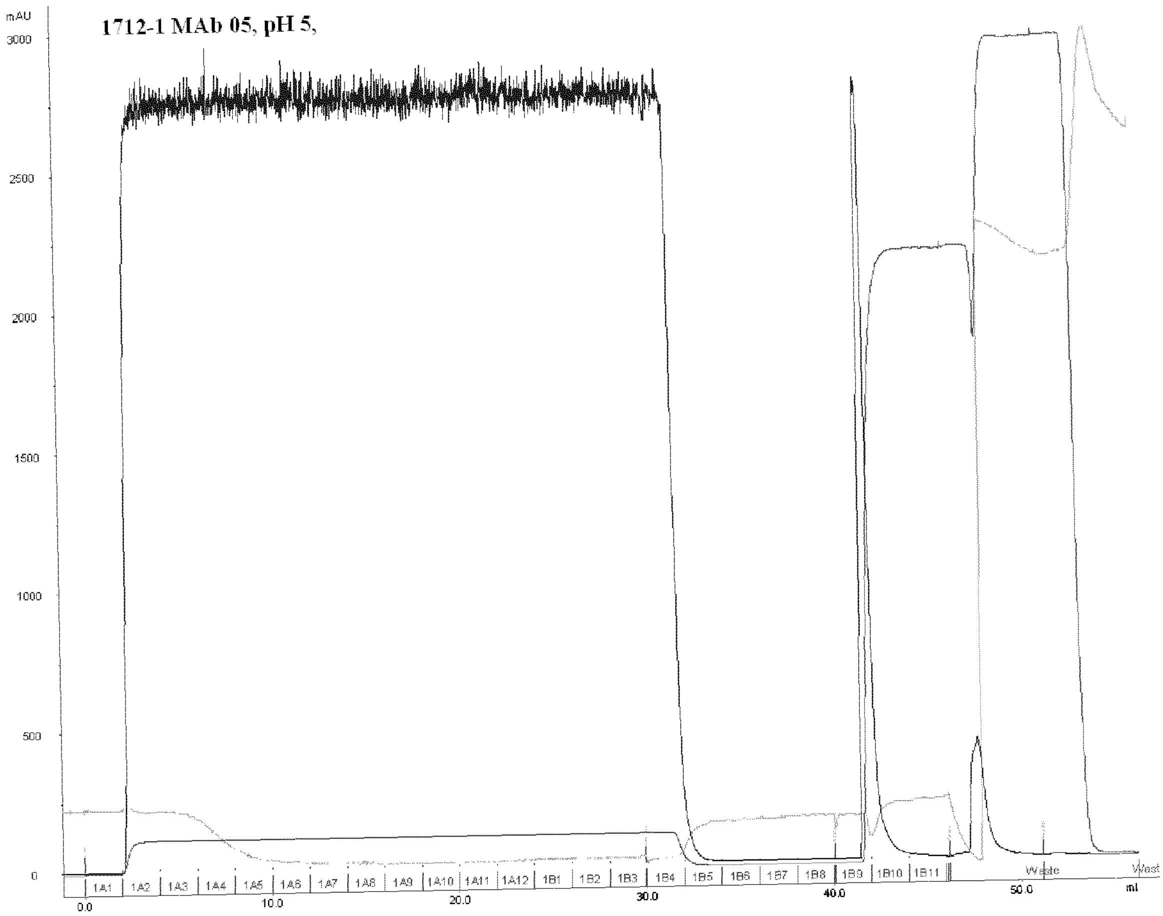
mAU
1712-1 MAb 05, pH 5,
3000
2500
2000
1500
1000
500
0
1A1
1A2
1A3
1A4
1A5
1A6
1A7
1A8
1A9
1A10
1A11
1A12
1B1
1B2
1B3
1B4
1B5
1B6
1B7
1B8
1B9
1B10
1B11
Waste
Wast
0.0
10.0
20.0
30.0
40.0
50.0
ml

Figure 17

Europäisches Patentamt
European Patent Office
Office européen des brevets

# EUROPEAN SEARCH REPORT

Application Number
EP 20 17 6384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIN ET AL: "Preparation of polymethacrylate monolithic stationary phases having bonded octadecyl ligands and sulfonate groups: Electrochromatographic characterization and application to the separation of polar solutes for pressurized capillary electrochromatography", JOURNAL OF CHROMATOGRAPHY A, ELSEVIER, AMSTERDAM, NL, vol. 1169, no. 1-2, 4 October 2007 (2007-10-04), pages 220-227, XP022284768, ISSN: 0021-9673, DOI: 10.1016/J.CHROMA.2007.08.061 * whole document, eg table 1 * ----- | 1,3-5,9 | INV. C07K1/16 B01D15/36 B01J20/26 B01J20/32 C07K1/18 C08F220/58 |
| X | DE 10 2006 061327 A1 (BASF CONSTR POLYMERS GMBH [DE]) 26 June 2008 (2008-06-26) * whole document, eg scheme 3 * ----- | 2,6-8 | |
| X | WO 2012/015908 A2 (MILLIPORE CORP [US]; YAVORSKY DAVID [US] ET AL.) 2 February 2012 (2012-02-02) * table 4, entries D, E, F * ----- | 10 | TECHNICAL FIELDS SEARCHED (IPC) C07K B01D B01J C08F |

-/--

The present search report has been drawn up for all claims

1

| Place of search | Date of completion of the search | Examiner |
|---|---|---|
| The Hague | 14 September 2020 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 17 6384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TANG ET AL: "Novel cell sheet carriers using polyion complex gel modified membranes for tissue engineering technology for cell sheet manipulation and transplantation", REACTIVE AND FUNCTIONAL POLYMERS, ELSEVIER, AMSTERDAM, NL, vol. 67, no. 11, 16 October 2007 (2007-10-16), pages 1388-1397, XP022300764, ISSN: 1381-5148, DOI: 10.1016/J.REACTFUNCTPOLYM.2007.07.058 * paragraph [0031] * ----- | 10 | |
| X | CENTOMO P ET AL: "Cross-linked poly-vinyl polymers versus polyureas as designed supports for catalytically active M^0 nanoclusters", JOURNAL OF MOLECULAR CATALYSIS A: CHEMICAL, ELSEVIER, AMSTERDAM, NL, vol. 300, no. 1-2, 2 March 2009 (2009-03-02), pages 48-58, XP026626175, ISSN: 1381-1169, DOI: 10.1016/J.MOLCATA.2008.10.039 [retrieved on 2008-11-05] * table 1 * ----- | 10 | |

TECHNICAL FIELDS SEARCHED (IPC)

1

The present search report has been drawn up for all claims

| Place of search | Date of completion of the search | Examiner |
|---|---|---|
| The Hague | 14 September 2020 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 17 6384

This annex lists the patent family members relating to the patent documents cited in the above-mentioned European search report. The members are as contained in the European Patent Office EDP file on
The European Patent Office is in no way liable for these particulars which are merely given for the purpose of information.

14-09-2020

| Patent document cited in search report | Publication date | Patent family member(s) | Publication date |
|---|---|---|---|
| DE 102006061327 A1 | 26-06-2008 | NONE | |
| WO 2012015908 A2 | 02-02-2012 | CN 103153423 A | 12-06-2013 |
| | | CN 104722101 A | 24-06-2015 |
| | | CN 108404453 A | 17-08-2018 |
| | | EP 2598223 A2 | 05-06-2013 |
| | | JP 6278929 B2 | 14-02-2018 |
| | | JP 6580168 B2 | 25-09-2019 |
| | | JP 2013535683 A | 12-09-2013 |
| | | JP 2015179099 A | 08-10-2015 |
| | | JP 2018059952 A | 12-04-2018 |
| | | KR 20130031351 A | 28-03-2013 |
| | | KR 20140119199 A | 08-10-2014 |
| | | KR 20150013940 A | 05-02-2015 |
| | | KR 20150023923 A | 05-03-2015 |
| | | KR 20150109499 A | 01-10-2015 |
| | | KR 20170058440 A | 26-05-2017 |
| | | KR 20180072865 A | 29-06-2018 |
| | | SG 186915 A1 | 28-02-2013 |
| | | SG 10201803960S A | 30-07-2018 |
| | | US 2012029176 A1 | 02-02-2012 |
| | | US 2015258540 A1 | 17-09-2015 |
| | | US 2018085743 A1 | 29-03-2018 |
| | | WO 2012015908 A2 | 02-02-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61609533
- US 61666578
- WO 2005044856 A
- US 6620918 B
- US 7427659 B
- US 8067182 B
- US 4944879 A
- US 4618533 A
- US 2009208784 A
- US 20100288690 A1
- US 20080257814 A1
- US 7118675 B
- US 61575349 B
- WO 2010098867 A
- WO 2010098867 PCT
- US 61575349


### Non-patent literature cited in the description


- **GIOVANNINI.** *Biotechnology and Bioengineering,* 2000, vol. 73, 522-529
- **JUNGBAUER.** *J. Chromatography,* 1989, vol. 476, 257-268
- **ALDINGTON et al.** *J. Chrom. B,* 2007, vol. 848, 64-78
- **LIU et al.** *J. Chrom. A.,* 2011, vol. 1218, 6943-6952
- Effects of stationary phase ligand density on high-performance ion-exchange chromatography of proteins. *J. Chrom.,* 1992, vol. 598, 7-13
- **FOGLE et al.** Effects of resin ligand density on yield and impurity clearance in preparative cation exchange chromatography. I. Mechanistic evaluation. *J. Chrom. A.,* 2012, vol. 1225, 62-69
- **SUDA et al.** Comparison of agarose and dextran-grafted agarose strong ion exchangers for the separation of protein aggregates. *J. Chrom. A,* 2009, vol. 1216, 5256-5264
- **GABRIELSON et al.** *J. Pharm. Sci.,* 2007, vol. 96, 268-279
- **STEIN et al.** *J. Chrom. B,* 2007, vol. 848, 151-158
- **ULBRICHT, M.** Advanced Functional Polymer Membranes. *Polymer,* 2006, vol. 47, 2217-2262
- **FOGLE et al.** *J. Chrom. A,* 2012, vol. 1225, 62-69
- Polymer Handbook. John Wiley & Sons, 1989, II
- **ODIAN.** J., Principles of Polymerization. John Wiley & Sons, 2004, 466
- Polymer Handbook. John Wiley and Sons, 1989, 11, , 156
- **BROOKS, C. A. ; CRAMER, S. M.** Steric mass-action ion exchange: Displacement profiles and induced salt gradients. *AIChE Journal,* 1992, vol. 38, 1969-1978
- **PEDERSEN.** Whey proteins as a model system for chromatographic separation of proteins. *J. Chromatogr. B,* 2003, vol. 790, 161-173
- **ANDERSON, M.J. ; WHITCOMB, P.J.** Design of Experiments. *Kirk-Othmer Encyclopedia of Chemical Technology,* 2010, 1-22